# FASCICULE DE BREVET EUROPEEN

(11) **EP 3 092 490 B1**
(45) Date de publication et mention de la délivrance du brevet: **22.05.2019**
(21) Numéro de dépôt: 15700961.4
(22) Date de dépôt: 06.01.2015
(51) Int. Cl.: G01N 33/50

(54) **PROCÉDÉ D'OBTENTION D'UN MODÈLE CELLULAIRE OU TISSULAIRE REPRÉSENTATIF D'UNE PEAU FRAGILE**
VERFAHREN ZUR HERSTELLUNG EINES FÜR EMPFINDLICHE HAUT REPRÄSENTATIVEN ZELLEN- ODER EINES GEWEBEMODELLS
METHOD FOR OBTAINING A CELL OR TISSUE MODEL REPRESENTATIVE OF DELICATE SKIN

(30) Priorité: 06.01.2014 FR 1450055
(43) Date de publication de la demande: 16.11.2016
(73) Titulaire: Pierre Fabre Dermo-Cosmétique, 92100 Boulogne-Billancourt (FR)
(72) Inventeur: GALLIANO, Marie-Florence, F-31700 Blagnac (FR); HERNANDEZ-PIGEON, Hélène, F-31270 Cugnaux (FR)
(74) Mandataire: Regimbeau
(86) Numéro de dépôt international: PCT/EP2015/050120
(87) Numéro de publication internationale: WO 2015/101677

(56) Documents cités:
- EP-A1- 1 457 780
- EP-A1- 1 635 176
- EP-A1- 1 964 546
- EP-A1- 2 019 133
- WO-A2-2015/014949
- FR-A1- 2 971 850
- US-A1- 2012 172 309
- DUPLAN H ET AL: "Effects of hydroxydecine(R) (10-hydroxy-2-decenoic acid) on skin barrier structure and function in vitro and clinical efficacy in the treatment of UV-induced xerosis", EUROPEAN JOURNAL OF DERMATOLOGY 2011 JOHN LIBBEY EUROTEXT FRA, vol. 21, no. 6, décembre 2011 (2011-12), pages 906-915, XP008172110, ISSN: 1167-1122
- HERNANDEZ-PIGEON H ET AL: "A new model of "fragile skin" in vitro on human keratinocytes and reconstructed epidermis", JOURNAL OF INVESTIGATIVE DERMATOLOGY, vol. 134, no. Suppl. 1, mai 2014 (2014-05) , page S64, XP002730023, ANNUAL MEETING OF THE SOCIETY-FOR-INVESTIGATIVE-DERMATOLOGY (SID); ALBUQUERQUE, NM, USA; MAY 07 -10, 2014
- STALDER J F ET AL: "Fragility of epidermis and its consequence in dermatology", JOURNAL OF THE EUROPEAN ACADEMY OF DERMATOLOGY AND VENEREOLOGY JUNE 2014 BLACKWELL PUBLISHING LTD GBR, vol. 28, no. SUPPL. 4, juin 2014 (2014-06) , pages 1-18, XP002730024, ISSN: 0926-9959
- ANDERHEGGEN B ET AL: "TAURINE IMPROVES EPIDERMAL BARRIER PROPERTIES STRESSED BY SURFACTANTS- A ROLE FOR OSMOLYTES IN BARRIER HOMEOSTASIS", JOURNAL OF COSMETIC SCIENCE, SOCIETY OF COSMETIC CHEMISTS, NEW YORK, NY, US, vol. 57, no. 1, 1 janvier 2006 (2006-01-01), pages 1-10, XP009070524, ISSN: 1525-7886
- D'ALESSANDRO M ET AL: "Keratin 14-null cells as a model to test the efficacy of gene therapy approaches in epithelial cells", JOURNAL OF INVESTIGATIVE DERMATOLOGY, NATURE PUBLISHING GROUP, US, vol. 131, no. 7, 1 juillet 2011 (2011-07-01), pages 1412-1419, XP002685849, ISSN: 1523-1747, DOI: 10.1038/JID.2011.19 [extrait le 2011-02-17]
- Terry L Riss et al.: "https://www.ncbi.nlm.nih.gov/books/NBK144 065/pdf/Bookshelf_NBK144065.pdf", , 1 July 2016 (2016-07-01), Retrieved from the Internet: URL:https://www.ncbi.nlm.nih.gov/books/NBK 144065/

## Description

### Résumé

La présente invention concerne un procédé pour obtenir un modèle cellulaire ou tissulaire qui reproduit des caractéristiques moléculaires et structurales d'une peau fragile. Ce modèle présente notamment toutes les caractéristiques d'une fonction barrière altérée. Ce modèle est obtenu en appliquant sur des cellules de l'épiderme ou sur un tissu cutané un double traitement (encore appelé « double-stress »). Ce double traitement induit la fragilisation desdites cellules ou dudit tissu cutané, reproduisant *in vitro* les caractéristiques d'une peau fragile. La présente invention concerne également une méthode de criblage ou d'évaluation de principes actifs pouvant avantageusement être utilisés pour prendre soin ou pour réparer des peaux fragiles.

### Contexte de l'invention

Une étude épidémiologique récente auprès de sujets adultes de différents pays (Haftek et al., 2013) a permis d'établir que la notion de « peau fragile » avait une résonance précise auprès des populations étudiées. Une « peau fragile » peut être définie comme une peau ayant une fragilité intrinsèque, de sorte qu'elle est plus réceptrice aux agressions de toutes sortes qu'une peau non fragile.

De façon générale, une peau fragile présente une perturbation de la fonction barrière entrainant une perméabilité et donc une réactivité plus importante aux stress ou aux agressions environnementales par rapport à une « peau normale » (non fragile). Ce type de peau est donc moins résistant aux stress ou aux agressions environnementales. Outre le dysfonctionnement de la fonction barrière, ces stress peuvent aussi entraîner une inflammation cutanée. Enfin, la peau fragile est aussi moins rapide à récupérer son état basal une fois exposée à de telles conditions.

Trois types de peaux fragiles peuvent être distingués :
- La peau fragile « constitutionnelle » : il s'agit d'une peau de bébé, de la peau d'une personne âgée, ou encore de la peau des zones fragiles du corps (paupières, visage ....)
- La peau fragile « environnementale » : il s'agit d'une peau qui a été agressée par un stress climatique (chaud, froid, sécheresse...), mécanique (frottements...), physique (UV, laser...) ou chimique (tensioactifs, solvants...),
- La peau fragile « pathologique » : il s'agit d'une peau subissant une dermatite atopique, une rosacée, de l'acné (la peau peut également être fragilisée par les traitements anti acnéiques)...

La composante inflammatoire se retrouve plus particulièrement dans le cas de peaux fragiles « environnementales » et « pathologiques ».

Toutes ces peaux fragiles demandent des soins particuliers afin de renforcer leurs propriétés naturelles de protection. C'est pourquoi, la recherche cosmétique et dermatologique s'attache à identifier des principes actifs qui, lorsqu'ils sont administrés par voie topique, sont capables de protéger et réparer ces peaux fragiles.

Duplan *et al.* décrit un modèle tissulaire de peau enflammée avec une fonction barrière altérée utilisé pour tester l'effet de l'hydroxydécine® (Duplan et al., Eur J Dermatol 2011; 21(6): 906-15).

Dans ce contexte, il serait très utile de disposer d'un modèle tissulaire et/ou cellulaire présentant toutes les caractéristiques d'une peau fragile, sur lequel ces principes actifs pourraient être testés en toute sécurité. Idéalement, un tel modèle devrait développer une réponse amplifiée des cellules ou des tissus aux stress, cette réponse amplifiée étant caractéristique de la réactivité des peaux fragiles. Ce modèle permettrait l'identification de composés ayant un effet certain et durable sur la réparation et la protection de ce type de peaux particulier. Ce modèle pourrait plus particulièrement être utilisé pour cribler des composés candidats et sélectionner ceux présentant un effet sur une peau fragile, ladite peau fragile étant caractérisée par une altération de l'intégrité structurale et/ou une réponse inflammatoire.

Pour répondre à ce besoin, les inventeurs ont donc cherché à mettre au point un modèle de peau fragilisée *in vitro* présentant une réponse amplifiée des cellules et/ou des tissus suite à une agression environnementale.

### Description détaillée de l'invention

Les inventeurs ont observé que l'application d'un double traitement (de nature chimique, déshydratante, mécanique ou physique) sur des cellules de l'épiderme ou sur un épiderme reconstruit permettait de reproduire *in vitro*, de façon artificielle, de nombreuses caractéristiques moléculaires et morphologiques des cellules présentes *in situ* dans les peaux fragiles.

En effet, ils ont constaté que l'application d'un premier traitement (ou stress) choisi parmi un stress chimique, déshydratant ou physique provoque une altération de la fonction barrière (qui se concrétise notamment par une diminution du marquage de la filaggrine et une désorganisation des jonctions serrées via l'altération du marquage de la claudine 1) et une augmentation de l'inflammation (notamment par la production de la cytokine IL8) dans des kératinocytes isolés et dans un épiderme reconstruit.

Les inventeurs ont également observé que l'application d'un premier traitement (ou stress) choisi parmi un stress chimique, déshydratant ou physique permet d'exacerber la réponse des cellules à un second stress chimique, déshydratant ou physique (par rapport aux cellules qui n'ont pas subi de premier stress et qui n'ont donc pas été fragilisées).

Les exemples 1 à 3 ci-dessous démontrent l'exacerbation de la réponse à un traitement déshydratant ou UV suite à un traitement au SDS.

Ainsi, l'application de ces deux traitements, permet de mimer de façon artificielle le phénotype d'une peau fragile.

Selon un premier aspect, la présente invention concerne un procédé *in vitro* ou *ex vivo* de fragilisation de cellules de l'épiderme, contenant au moins deux traitements choisis parmi : un traitement chimique, un traitement déshydratant réalisé en plaçant lesdites cellules dans un compartiment contenant une humidité comprise entre 5 et 40% et ayant une température comprise entre 30 et 40°C, un traitement physique et un traitement mécanique, caractérisé en ce que ledit traitement chimique est réalisé en mettant en contact lesdites cellules avec des détergents ou des cétones, en ce que ledit traitement physique est une irradiation UV et en ce que ledit traitement mécanique est une compression ou une traction.

Ce procédé peut être réalisé *in vitro* (sur des cellules isolées de l'épiderme, par exemple des kératinocytes humains ; ou sur des lignées cellulaires ; ou sur des épidermes reconstruits générés à partir desdites cellules isolées) ou *ex vivo* (sur des cellules au sein d'un tissu, par exemple d'un explant de peau, sans contact avec l'organisme qui l'a généré).

Au sens de la présente invention, un « modèle de peau fragilisée » désigne une population de cellules isolées de l'épiderme ou un tissu contenant des cellules de l'épiderme, lesdites cellules ou ledit tissu présentant des caractéristiques d'une fonction barrière altérée (caractérisée notamment par une altération de l'intégrité structurale et/ ou une réponse inflammatoire).

Par « fonction barrière», on entend, au sens de la présente invention, la protection conférée par la couche épidermique sur la peau. Cette « fonction » consiste plus particulièrement à protéger l'organisme de la déshydratation dans un milieu non aquatique. Les paramètres qui peuvent être mesurés *in vitro* pour évaluer l'intégrité de cette barrière cutanée sont, entre autres, les protéines des jonctions serrées (dont la claudine -1), la filaggrine et de manière plus générale des marqueurs de l'enveloppe cornée (dont la loricrine), et également la résistance électrique transépithéliale.

Par « fonction barrière altérée », on entend, au sens de la présente invention, une diminution de la fonction de protection telle que définie ci-dessus.

Les peaux fragiles présentent de nombreux signes d'une fonction barrière altérée (Ramos e Silva M. et al, 2012 ; Biniek K. et al, 2012)De préférence, les cellules de ladite population ou dudit tissu présentent au moins une, de préférence deux et de manière encore plus préférée trois caractéristiques choisies parmi :
i) Un marquage altéré de la filaggrine (qui est un marqueur des kératinocytes différenciés participant à l'agrégation des tonofilaments de kératine et à la formation du facteur naturel d'hydratation),
ii) Un marquage altéré de la claudine-1 (qui est une protéine des jonctions serrées),
iii) Une résistance transépithéliale diminuée (altération des jonctions serrées),
iv) Une altération de la morphologie cellulaire (cytosquelette d'actine),
v) Une expression augmentée de cytokines inflammatoires, telles que l'IL1α ou l'IL8,
vi) Une quantité augmentée de LDH (lactate déshydrogénase) dans les surnageants cellulaires,
lesdits paramètres étant comparés aux valeurs mesurées dans des cellules d'une peau normale, saine.

L'expression de filaggrine ou de claudine-1 peut être analysée notamment par immunohistochimie au moyen d'anticorps dirigés spécifiquement contre ces protéines. Cette technique permet également d'apprécier la localisation de ces protéines et d'identifier une éventuelle désorganisation de leur répartition.

L'intégrité (et donc la résistance) des jonctions serrées peut quant à elle être analysée par la mesure de la résistance électrique trans-épithéliale (TEER), au moyen d'un ohmmètre.

La morphologie cellulaire peut être analysée notamment par immunohistochimie au moyen d'anticorps dirigés spécifiquement, par exemple, contre l'actine. Cette technique permet également d'apprécier le cytosquelette et d'identifier une éventuelle désorganisation.

L'expression des cytokines inflammatoires peut être mesurée par exemple par analyse de l'expression des gènes codant pour lesdites cytokines (par PCR en temps réel), ou par le dosage desdites cytokines produites (par ELISA, par exemple).

Enfin, la quantité de LDH au sein des cellules peut être évaluée par dosage colorimétrique. Des kits commerciaux (Abcam, Pierce, etc.) peuvent être utilisés à cet effet.

Le modèle cellulaire de peau fragilisée correspond à une population de cellules de l'épiderme isolées (c'est-à-dire non incluses dans un tissu) présentant une à six des caractéristiques moléculaires ci-dessus énoncées. Dans un mode de réalisation préféré, ladite population de cellules présente deux, trois, quatre, cinq ou six des caractéristiques moléculaires ci-dessus énoncées. Dans un mode de réalisation encore plus préféré, ladite population de cellules présente une à cinq des caractéristiques i), ii), iv), v), et vi) ci-dessus énoncées.

Le modèle tissulaire de peau fragilisée correspond quant à lui à un tissu cutané, naturel (explant, *ex vivo*) ou reconstruit (*in vitro*), contenant des cellules de l'épiderme qui sont reliées les unes aux autres pour former un matériau solide, ces cellules présentant une à six des caractéristiques moléculaires i) à vi) ci-dessus énoncées. Dans un mode de réalisation préféré, ledit tissu présente deux, trois, quatre, cinq ou six des caractéristiques moléculaires i) à iv) ci-dessus énoncées.

Les « cellules de l'épiderme » auxquelles il est fait référence dans la présente demande sont des kératinocytes. Il est possible d'obtenir des cellules isolées de l'épiderme par toute technique classique connue de l'homme du métier, par exemple à partir d'explants de peau humains. Ces cellules peuvent être cultivées dans des milieux classiques commerciaux (tel que ceux utilisés par les inventeurs dans l'exemple 1 ci-dessous), dans des conditions de culture classiques (37°C, 5% CO₂, 98% d'humidité).

Le terme « tissu cutané» désigne dans la présente invention un explant, une peau reconstruite ou un épiderme reconstruit, de préférence humain, qui contient des cellules différenciées, réparties en plusieurs couches stratifiées.

Par « explant », on entend au sens de la présente invention, une biopsie *ex vivo* de peau humaine issue de déchets opératoires, qui comprend la totalité de l'épiderme et un compartiment dermique.

Par « peau reconstruite », on entend au sens de la présente invention un matériau contenant une composante épidermique (kératinocytes) et une composante dermique (fibroblastes), les deux composantes ayant été générées *in vitro.* La peau reconstruite est disponible commercialement.

Par « épiderme reconstruit », on entend au sens de la présente invention un épiderme généré *in vitro* par des techniques classiques bien connues de l'homme du métier (cf., par exemple, Limat et al., Cells Tissues Organs 2002; Poumay Y. et al., ArchDermatolRes. 2004). Lorsque ce traitement est appliqué à des cellules isolées (modèle cellulaire), ledit traitement par rayonnement UV est de préférence un rayonnement UVB ou un rayonnement solaire. Dans un mode de réalisation particulier de l'invention, la dose d'UVB est comprise entre 80 et 150 mJ/cm² d'UVB ; et de préférence la dose d'UVB est de 120 mJ/cm² d'UVB.

Plusieurs traitements de nature différente ou identique peuvent être appliqués sur les deux modèles ci-dessus décrits. Par « nature d'un traitement », on entend ici la caractérisation de ce traitement en quatre catégories (chimique, déshydratante, mécanique ou physique). Ainsi, les termes « traitements différents » ou « traitements de nature différente » sont ici synonymes, et désignent, dans le cadre de la présente invention, des traitements de catégorie (chimique, déshydratante, mécanique ou physique) différente (un traitement chimique et un traitement mécanique, un traitement chimique et un traitement déshydratant, etc.).

A l'inverse, les termes « traitements identiques » ou « traitements de nature identique », ici synonymes, désignent des traitements appartenant à la même catégorie (chimique, déshydratante, mécanique ou physique : par exemple deux traitements chimiques, même si le composé chimique utilisé n'est pas le même pour les deux traitements).

Le traitement chimique auquel peuvent être soumises les cellules de l'épiderme (isolées ou au sein d'un tissu, comme indiqué plus haut) est également appelé « stress chimique » dans le cadre de la présente demande. Ce traitement consiste à mettre lesdites cellules en contact avec un agent chimique agressif capable de les fragiliser efficacement, sans toutefois les détruire. Ledit agent chimique est choisi parmi les détergents (tels que le SDS) ou encore les cétones (tels que l'acétone). Les détergents peuvent être utilisés sur les cellules isolées ou sur les tissus cutanés. En revanche, il est préférable de ne pas utiliser les cétones sur des cellules isolées.

Cet agent chimique peut par exemple être ajouté dans un milieu de culture classiquement utilisé pour maintenir les cellules kératinocytes en culture. Le milieu utilisé dans le modèle cellulaire est typiquement du Keratinocyte-Serum Free (K-SFM), éventuellement complémenté en sérum. Le milieu utilisé dans le modèle tissulaire est typiquement du DMEM, éventuellement supplémenté en insuline, transferrine, et/ou en sélénium. Ledit milieu est ensuite mis en contact des cellules : dans le cas de cellules isolées (modèle cellulaire), les cellules peuvent être suspendues dans le milieu contenant l'agent chimique. Pour un tissu cutané (modèle tissulaire), ledit milieu peut être ajouté sur et/ou placé sous ledit tissu ; il est de préférence appliqué à la surface de l'épiderme. L'application de ce traitement a lieu dans des conditions de culture classiques (par exemple, 37°C, 5% CO₂, 98% d'humidité).

La concentration dudit agent chimique doit être choisie de façon à ce que les cellules de l'épiderme soient fragilisées, sans toutefois être détruites (le double traitement ne doit pas, *in fine,* être cytotoxique). De même, la durée du traitement doit être adaptée dans cette même perspective. Ladite concentration et ladite durée dépendent bien entendu de la nature dudit agent chimique, et du modèle considéré (cellulaire ou tissulaire).

Dans un mode de réalisation préféré, ledit agent chimique est le Sodium Dodécyl Sulfate (SDS).

Dans ce cas, une concentration comprise entre 5 et 30 µg/mL, de préférence entre 10 et 15µg/mL peut être utilisée à des cellules de l'épiderme isolées (modèle cellulaire). Par ailleurs, une concentration comprise entre 1 et 1,5 mg/mL peut être utilisée sur un tissu cutané (modèle tissulaire). La durée de ce traitement peut varier de 30 minutes à 4 heures pour le modèle cellulaire. Elle peut être beaucoup plus longue (entre 2h et 24h, de préférence 18h) pour le modèle tissulaire. Typiquement, le traitement chimique au SDS dure une heure lorsque ce détergent est appliqué sur des cellules isolées (modèle cellulaire) et 18 heures lorsqu'il est appliqué sur un tissu cutané.

Plus précisément, il est possible d'utiliser le SDS :
- Sur des cellules isolées (modèle cellulaire) : à une concentration comprise entre 10 et 15 µg/mL pendant une heure,
- Sur un tissu cutané (modèle tissulaire) : à une concentration comprise entre 1 et 1,5 mg/mL pendant 18 heures.

Le traitement déshydratant auquel peuvent être soumises les cellules de l'épiderme (isolées ou au sein d'un tissu, comme indiqué plus haut) peut être réalisé avant ou après un autre traitement (chimique, physique ou mécanique) ; il est réalisé de préférence après le traitement chimique au SDS. Ce traitement déshydratant consiste à placer lesdites cellules (isolées ou au sein d'un tissu cutané) dans des conditions thermiques et hydrométriques telles qu'elles subissent une déshydratation partielle. Les conditions de cette étape de déshydratation sont choisies de façon telle qu'elle altère la fonction barrière et donc fragilise les cellules, sans toutefois entraîner la mort cellulaire.

Dans un mode de réalisation préféré, ce traitement déshydratant est réalisé en plaçant lesdites cellules ou lesdits tissus cutanés dans un compartiment où la température et l'humidité de l'air sont contrôlables. Cette température doit être suffisamment chaude pour induire la déshydratation, sans toutefois affecter la viabilité des cellules. Une température comprise entre 30°C et 40°C, de préférence de 37°C, sera utilisée pour les deux types de modèles. En parallèle, le taux d'humidité devra être inférieur à celui utilisé classiquement dans les incubateurs (98%). Un taux d'humidité compris entre 5 et 40%, de préférence entre 10% et 30%, sera utilisé pour les deux types de modèles.

Lorsque ce traitement est appliqué à des cellules isolées (modèle cellulaire) ledit traitement déshydratant est de préférence maintenu pendant une durée comprise entre 10 minutes et 40 minutes. De manière encore plus préférée, cette durée est comprise entre 20 et 25 minutes.

Lorsque ce traitement est appliqué à un tissu cutané (modèle tissulaire), ledit traitement déshydratant est de préférence maintenu pendant une durée comprise entre 30 minutes et 2 heures. De manière encore plus préférée, cette durée est comprise entre 45 minutes et 80 minutes. De manière préférée entre toute, cette durée est de 60 minutes (1h).

Le traitement mécanique auquel peuvent être soumises les cellules de l'épiderme (isolées ou au sein d'un tissu, comme indiqué plus haut) peut être réalisé avant ou après un autre traitement (chimique, physique ou déshydratant). Ce traitement consiste à appliquer sur ces cellules une compression ou une traction.

Le traitement physique auquel peuvent être soumises les cellules de l'épiderme (isolées ou au sein du tissu, comme indiqué plus haut) peut être réalisé avant ou après un autre traitement (chimique, mécanique ou déshydratant). Un traitement par rayonnement UV est réalisé en irradiant les cellules avec des UV A, des UV B ou un rayonnement UV solaire dans des conditions bien connues de l'homme du métier (cf. notamment Iordanov M.S. et al, J. Biol. Chem. 1998).

Dans le procédé de l'invention, les traitements appliqués aux cellules de l'épiderme sont de nature différente. Un traitement chimique peut être combiné à un traitement déshydratant, un traitement physique ou à un traitement mécanique. Par ailleurs, un traitement déshydratant peut être combiné à un traitement chimique, un traitement physique ou à un traitement mécanique. Dans un mode de réalisation très particulier, trois traitements différents (chimique, physique et déshydratant) sont appliqués sur les cellules de l'épiderme. L'étape de fragilisation peut être la résultante d'une combinaison de deux stress.

Dans un mode de réalisation préféré, les cellules de l'épiderme des modèles de l'invention ont subi deux traitements. Ces deux traitements sont de préférence un traitement chimique et un traitement déshydratant ou un traitement chimique et un traitement physique.

Dans ce mode de réalisation, l'ordre des deux traitements (par exemple chimique et déshydratant ou physique) n'a pas d'importance. En d'autres termes, le traitement déshydratant ou physique peut être réalisé avant le traitement chimique, et inversement. Comme démontré dans les exemples ci-dessous, c'est en effet l'application d'un « double stress » qui entraîne la fragilisation adéquate des cellules et ces deux traitements peuvent se compléter et se potentialiser mutuellement (fragilisant les cellules / tissus de façon synergique). Dans un mode de réalisation préféré, cependant, ledit traitement chimique est réalisé avant ledit traitement déshydratant ou physique.

Les traitements (au moins deux) auxquels sont soumises les cellules (isolées ou au sein d'un tissu) dans le procédé de l'invention peuvent être espacés dans le temps de 4h à 30h, de préférence de 6h à 30h, de manière encore plus préférée de 10h à 30h, et de manière préférée entre toutes de 18h à 30h. Typiquement, pour des raisons pratiques, les traitements peuvent être espacés de 18h à 24h ou de 24h. Entre ces traitements, les modèles (cellulaires et tissulaires) sont cultivés dans des milieux adéquats tels que ceux décrits ci-dessus, dans des conditions de culture classiques (37°C, 5% CO₂, 98% d'humidité).

Il est utile de rappeler ici que le modèle cellulaire ou tissulaire de peau fragilisée correspond :
- à une population de cellules de l'épiderme isolées (c'est-à-dire non incluses dans un tissu),
- ou à un tissu cutané, naturel ou reconstruit, contenant des cellules de l'épiderme qui sont reliées les unes aux autres pour former un matériau solide ;
ces cellules présentant une à six des caractéristiques moléculaires i) à vi) suivantes :
i) Un marquage altéré de la filaggrine,
ii) Un marquage altéré de la claudine-1,
iii) Une résistance transépithéliale diminuée,
iv) Une désorganisation du cytosquelette d'actine,
v) Une expression augmentée de cytokines inflammatoires, telles que l'IL1α ou l'IL8,
vi) Une quantité augmentée de LDH dans les surnageants cellulaires,
lesdits paramètres étant comparés aux valeurs mesurées dans des cellules d'une peau normale, saine.

Le tissu cutané peut par exemple être un explant, une peau reconstruite ou un épiderme reconstruit.

Dans un dernier aspect, la présente invention vise une méthode de criblage pour l'identification *in vitro* ou *ex vivo* de composés destinés au soin ou à la protection des peaux fragiles, comprenant la mise en contact avec le modèle cellulaire ou tissulaire obtenu par le procédé de la présente invention, par exemple par application topique desdits composés (ou des compositions les contenant) ou par ajout desdits composés dans le milieu de culture. Le composé candidat sera sélectionné si, en sa présence, on observe une restauration de la fonction barrière.

Ces composés peuvent être avantageusement inclus dans une composition, par exemple dans une composition cosmétique ou dermatologique (cf. exemple II ci-dessous).

Ces méthodes de criblage comprennent une à six étape(s) i) à vi) ci-dessous:
i) Mesurer l'expression et/ou la localisation de la filaggrine, et/ou
ii) Mesurer l'expression et/ou la localisation de la claudine-1, et/ou
iii) Mesurer l'intégrité et/ou la résistance des jonctions serrées, et/ou
iv) Analyser la morphologie cellulaire (cytosquelette d'actine), et/ou
v) Mesurer l'expression de cytokines inflammatoires, telles que l'IL1α ou l'IL8, et/ou
vi) Mesurer la quantité de LDH (lactate déshydrogénase) dans les surnageants cellulaires,
ainsi que la comparaison de la ou des valeur(s) obtenues avec celle(s) mesurée(s) en l'absence du composé candidat.

Des techniques de mesure de ces différentes caractéristiques ont été citées plus haut. Ces techniques sont classiques pour l'homme du métier, de sorte qu'il n'est pas utile de les détailler ici.

Le composé candidat peut être appliqué sur les cellules ou le tissu avant que l'ensemble des traitements soient réalisés, entre les traitements, ou après que l'ensemble des traitements ont été réalisés.

Le composé candidat (ou une composition le contenant) sera sélectionné si, en sa présence, on observe une restauration de la fonction barrière, par exemple si on observe:
i) Une restauration du marquage de la filaggrine, et/ou
ii) Une restauration du marquage de la claudine-1, et/ou
iii) Une résistance transépithéliale restaurée, et/ou
iv) Une organisation du cytosquelette d'actine restaurée, et/ou
v) Une expression diminuée de cytokines inflammatoires, telles que l'IL1α ou l'IL8, et/ou
vi) Une quantité diminuée de LDH (lactate déshydrogénase) dans les surnageants cellulaires,
par rapport aux valeurs mesurées en l'absence de ce composé candidat.

Est décrite par ailleurs une méthode de criblage pour l'identification *in vitro* ou *ex vivo* de composés destinés au soin ou à la protection des peaux fragiles ou à restaurer la fonction barrière de la peau, comprenant les étapes suivantes:
a. Obtenir une population de cellules de l'épiderme,
b. Appliquer au moins un traitement chimique, un traitement mécanique, un traitement physique ou un traitement déshydratant auxdites cellules,
c. Appliquer un composé candidat dans leur milieu de culture,
d. Appliquer auxdites cellules au moins un traitement chimique, un traitement mécanique, un traitement physique ou un traitement déshydratant, ledit traitement étant identique ou différent de celui appliqué à l'étape b),
e. Mesurer un à six paramètre(s) choisi(s) parmi :
   i. l'expression et/ou la localisation de la filaggrine,
   ii. l'expression et/ou la localisation de la claudine-1,
   iii. l'intégrité et/ou la résistance des jonctions serrées,
   iv. l'organisation du cytosquelette d'actine,
   v. l'expression de cytokines inflammatoires, telles que l'IL1α ou l'IL8,
   vi. la quantité de LDH (lactate déshydrogénase) dans les surnageants cellulaires,
f. Comparer la ou les valeur(s) obtenue(s) à l'étape e) avec celle(s) mesurée(s) en l'absence du composé candidat.

Une telle méthode de criblage permet d'identifier des composés capables de prévenir la fragilisation de la peau.

Dans un mode de réalisation alternatif, les étapes b) et c) mentionnées ci-dessus sont réalisées dans l'ordre inverse, c'est-à-dire, que le composé candidat est appliqué sur les cellules avant que l'ensemble des traitements soient réalisés. Une telle méthode de criblage permet d'identifier des composés capables de prévenir la fragilisation de la peau.

Dans un mode de réalisation alternatif, les étapes c) et d) mentionnées ci-dessus sont réalisées dans l'ordre inverse, c'est-à-dire, que le composé candidat est appliqué sur les cellules après que le deuxième traitement a été réalisé. Une telle méthode de criblage permet d'identifier des composés capables de réparer une peau fragilisée.

Est aussi décrite une méthode de criblage pour l'identification *in vitro* ou *ex vivo* de composés destinés au soin ou à la protection des peaux fragiles ou à restaurer la fonction barrière de la peau, comprenant les étapes suivantes:
a. Obtenir un tissu cutané,
b. Appliquer au moins un traitement chimique, un traitement mécanique, un traitement physique ou un traitement déshydratant audit tissu cutané,
c. Appliquer un composé candidat dans son milieu de culture ou appliquer une composition contenant ce composé candidat de façon topique,
d. Appliquer audit tissu cutané au moins un traitement chimique, un traitement mécanique, un traitement physique ou un traitement déshydratant, ledit traitement étant identique ou différent de celui appliqué à l'étape b),
e. Mesurer un à six paramètre(s) choisi(s) parmi :
   i. l'expression et/ou la localisation de la filaggrine ,
   ii. l'expression et/ou la localisation de la claudine-1,
   iii. l'intégrité et/ou la résistance des jonctions serrées,
   iv. l'organisation du cytosquelette d'actine,
   v. l'expression de cytokines inflammatoires, telles que l'IL1α ou l'IL8,
   vi. la quantité de LDH (lactate déshydrogénase) dans les surnageants cellulaires,
f. Comparer la ou les valeur(s) obtenue(s) à l'étape e) avec celle(s) mesurée(s) en l'absence du composé candidat.

Une telle méthode de criblage permet d'identifier des composés capables de prévenir la fragilisation de la peau.

Dans un mode de réalisation alternatif, les étapes b) et c) mentionnées ci-dessus sont réalisées dans l'ordre inverse, c'est-à-dire, que le composé candidat est appliqué sur le tissu avant que l'ensemble des traitements soient réalisés. Une telle méthode de criblage permet d'identifier des composés capables de prévenir la fragilisation de la peau.

Dans un mode de réalisation alternatif, les étapes c) et d) mentionnées ci-dessus sont réalisées dans l'ordre inverse, c'est-à-dire, que le composé candidat est appliqué sur le tissu après que le deuxième traitement a été réalisé. Une telle méthode de criblage permet d'identifier des composés capables de réparer une peau fragilisée.

Dans toutes ces méthodes de criblage, la quantité des protéines sécrétée peut être mesurée par toute technique de dosage protéique connue de l'homme de l'art, et notamment par ELISA ou immunomarquages ou par western-blot. Par ailleurs, l'expression des protéines filaggrine, claudine-1, IL1α et IL8 peut être évaluée en mesurant la quantité d'ARNm codant pour ces protéines, par qPCR, par Northern Blot.

### Légendes des figures

**Figure 1****.** Schéma de la réalisation du double stress et des contrôles équivalents simples stress : modèle cellulaire (A) et modèle tissulaire (B).
**Figure 2****.** Immunomarquage des cellules traitées par le doubles stress SDS 10µg/mL + déshydratation 20 minutes à T0h par rapport à des cellules témoin et des cellules traitées uniquement avec les simples stress équivalents (SDS 10µg/mL T24h et déshydratation 20 minutes T0h), obj x40. A. Marquage de la claudine 1. B. Marquage de l'actine. C. Marquage de la filaggrine. Observation microscopique réalisée contre témoin négatif (anticorps secondaire seul). Expériences représentatives de trois expériences indépendantes.
**Figure 3****.** Quantité Relative d'ARNm de IL8 4h après un double stress par rapport à des cellules témoin et des cellules contrôles simples stress équivalents. QR d'une expérience représentative de 4 expériences obtenues 4h après un double stress SDS 10 µg/mL + déshydratation 20 minutes.
**Figure 4****.** Résultats du dosage la LDH, à partir des surnageants de culture récupérés 18h après le deuxième stress ou 18h après les simples stress, exprimé en ratio par rapport aux tissus non traités, n=3 sur 3 à 5 épidermes.
**Figure 5****.** Coupes histologiques d'un épiderme traité avec le simple stress SDS (à gauche) ou le double stress de l'invention (à droite).
**Figure 6****.** Résultats du dosage de l'IL-8 (A et D) et de l'IL-1α (B et C), à partir des surnageants de culture récupérés 18h (A et B) ou 36h (C et D) après le deuxième stress ou 18h (A et B) ou 36h (C et D) après les simples stress, exprimé en ratio par rapport aux tissus non traités, n=3 sur 3 épidermes.
**Figure 7****.** Mesure de la TEER sur des épidermes non traités, à la fin du simple stress SDS, à la fin du simple stress de déshydratation (désh.), ou après le double-stress de l'invention. n=3 sur 3 épidermes.
**Figure 8****.** Immunomarquage de la filaggrine sur des épidermes non traités (à gauche) et traités au simple stress SDS (à droite) pendant 18h.
**Figure 9****.** Marquage de la claudine 1 sur des cellules ayant subi un double-stress (SDS 10µg/mL + déshydratation 20 minutes) à T0h traités ou pas avec l'actif C à 100 µg/mL par rapport aux cellules témoins n'ayant pas subi de double stress (non traitées) et aux cellules traitées avec le contrôle positif (tréhalose, 10µM), obj x40. Observation microscopique réalisée par rapport au témoin négatif (anticorps secondaire seul). Expérience représentative de 3 expériences indépendantes.
**Figure 10****.** Marquage de l'actine par la phalloïdine sur des cellules ayant subi un double stress (SDS 10µg/mL + déshydratation 20 minutes) à T0h traités ou pas avec l'actif C à 100 µg/mL par rapport aux cellules témoins n'ayant pas subi de double stress (non traitées) et aux cellules traitées avec le contrôle positif (tréhalose, 10µM), obj x40. Observation microscopique réalisée par rapport au témoin négatif (anticorps secondaire seul). Expérience représentative de 3 expériences indépendantes.
**Figure 11****.** Marquage de la Filaggrine sur des cellules ayant subi un double stress (SDS 10µg/mL + déshydratation 20 minutes) à T0h traités par l'actif C à 100 µg/mL par rapport aux cellules témoins n'ayant pas subi de double stress (non traitées) et aux cellules traitées avec le contrôle positif (tréhalose, 10µM), obj x40. Observation microscopique réalisée contre témoin négatif (anticorps secondaire seul). Expérience représentative de 3 expériences indépendantes.
**Figure 12****.** Expression génique de IL8 dans l'expérience de valorisation de l'actif C à 10 et 100 µg/mL. Quantité relative d'ARNm obtenu 4h après le second stress de déshydratation, par rapport au témoin non traité, au témoin double stress (SDS 10µg/mL + déshydratation 20 minutes) et au contrôle positif, le tréhalose à 10 µM. Expérience représentative de deux expériences indépendantes.
**Figure 13****.** Résultats du dosage de l'activité de la LDH, à partir des surnageants des épidermes traités ou non par le double stress +/- formulations, n=2 essais sur 3 épidermes chacun. Expérience représentative de trois essais indépendants sur trois épidermes.
**Figure 14****.** Résultats du dosage d'IL-1α, à partir des surnageants des épidermes traités ou non par le double stress +/- formulations, expérience représentative de trois essais indépendants sur 3 épidermes.
**Figure 15****.** Immunomarquage de la filaggrine sur des épidermes non traités (en haut, à gauche), ayant subi un double stress (en haut, à droite), en présence de la préformule placébo (en bas à gauche) ou contenant l'extrait de plantules d'avoine (en bas à droite). Expérience représentative de deux essais indépendants.
**Figure 16****.** Immunomarquage de la Claudine-1 sur des épidermes non traités (en haut, à gauche), ayant subi un double stress (en haut, à droite), en présence de la préformule placébo (en bas à gauche) ou contenant l'extrait de plantules d'avoine (en bas à droite). Expérience représentative de deux essais indépendants.
**Figure 17****.** Schéma du mode opératoire de la valorisation d'actifs sur le modèle cellulaire double stress.
**Figure 18****.** Expression génique d'IL8 après un double stress « déshydratation puis SDS » (quantités relatives d'ARNm obtenues 4h après le double stress, moyenne de n=4).
**Figure 19****.** Expression génique d'IL8 et IL1alpha après le simple stress SDS, le simple stress déshydratation et le double stress, à 36h à partir du début des incubations.
**Figure 20****.** Schéma du mode opératoire de la valorisation d'actifs sur le modèle tissulaire double stress.
**Figure 21****.** Schéma de la réalisation du double stress et des contrôles équivalents simples stress SDS et UV totaux sur le modèle cellulaire
**Figure 22****.** Immunomarquage des cellules traitées par le double stress SDS 10µg/mL + UV totaux à 1.65 J/cm² à T1h par rapport à des cellules témoin et des cellules traitées uniquement avec les simples stress équivalents (SDS 10 µg/mL T25h et UV totaux à 1.65 J/cm² à T1h), obj x40. Marquage de l'actine. Expériences représentatives de trois expériences indépendantes.
**Figure 23****.** Quantité Relative d'ARNm de LOR, CNFN (A) et CXCL14 (B) 24h après un double stress par rapport à des cellules témoin et des cellules contrôles simples stress équivalents. Moyenne des QR de 8 expériences obtenues 24h après un double stress SDS 10 µg/mL + UV totaux à 1.65 J/cm².

### Exemples

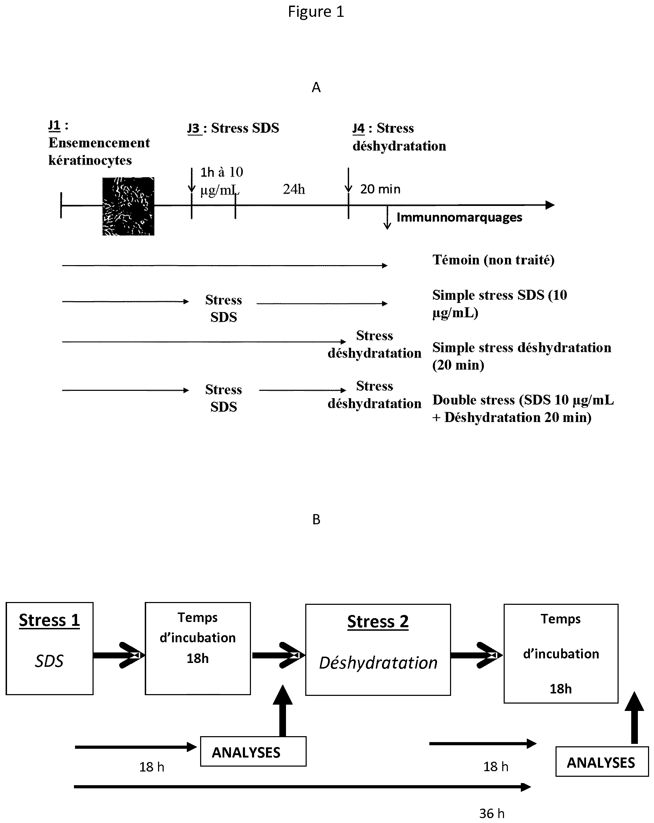

### Exemple 1 : Caractérisation du modèle cellulaire représentatif de peaux fragilisées (Figure 1A)

### 1.1. Matériels et Méthodes

### 1.1.1. Matériel Biologique

La culture cellulaire a été réalisée à partir de kératinocytes normaux humains (KNH) issus d'explants de peau (abdominoplastie ou diminution mammaire). Les KNH ont été cultivés en condition stérile avec du milieu Keratinocyte-Serum Free Medium (K-SFM) (Gibco® - Life Technologies™) complémenté avec du Bovine PituitaryExtract (BPE) et de l'EpidermalGrowth Factor Human recombinant (EGF) (Gibco®- Life Technologies™). Le milieu a été complémenté en antibiotique par de la Primocin™ (InvivoGen). La lignée de kératinocytes HaCaT a été cultivée en condition stérile avec du milieu DMEM *(Dulbecco'sModifiedEagle's Medium)* complémenté avec 5% de sérum de veau foetal (Gibco®-Life Technologies™). Les cellules ont été cultivées dans un incubateur à 37 °C sous atmosphère saturée en eau à 5 % de CO₂.

### 1.1.2. Modèle de cellules fragilisées

### 1.1.2.1. Double-stress : SDS puis déshydratation

Le premier stress avec SDS a été réalisé par une incubation des cellules 1 heure à 37 °C à la concentration souhaitée de SDS (Sigma-Aldrich). A la suite du stress, les cellules ont été rincées au Dulbecco's Phosphate Buffered Saline (1X) [-] Cacl₂ [-] MgCl₂ (D-PBS) (Gibco®-Life Technologies™) et remises en culture au temps T₀ avec du K-SFM sans compléments. 24 heures après le premier stress (SDS) les cellules ont subi un second stress différent du premier (déshydratation).

Les cellules ont été déshydratées dans une enceinte climatique (Vötsch - Industrietechnik) à 37°C et 15 % d'humidité pendant le temps souhaité. Les cellules traitées étaient dépourvues de tout liquide au moment de la déshydratation, tandis que les cellules témoins étaient dans du tampon Hanks' Balanced Salt Solution (1X) [+] CaCl₂ [+] MgCl₂ (HBSS) (Gibco® - Invitrogen™) remplaçant le milieu de culture. Après déshydratation les cellules ont été remises en culture au temps T₀ avec du K-SFM sans compléments.

### 1.1.2.2. Double stress : déshydratation puis SDS

Un double stress a été appliqué sur des cellules de la lignée HaCaT. Ces cellules ont subi un premier stress thermique (déshydratation pendant 20 min à 15% d'humidité). Après récupération de 24h, les cellules ont été soumises à un deuxième stress chimique (SDS à 15 µg/ml pendant 1h) pour analyser l'expression de différents ARNm.

### 1.1.3. Caractérisation du modèle par analyse protéique

Juste après le 2^{ème} stress, les cellules ont été fixées avec une solution d'acétone à - 20 °C pendant 10 minutes. Avant l'immunomarquage les cellules ont été réhydratées avec du D-PBS, puis les membranes cellulaires ont été perméabilisées avec du Triton® X-100 (Sigma-Aldrich) à 0,1 % pendant 15 minutes. Après plusieurs rinçages avec du D-PBS, les sites spécifiques ont été saturés avec de l'Albumine de Sérum Bovin (BSA) (Sigma® Life science) à 3 % pendant 15 minutes. Puis, les anticorps primaires ont été ajoutés dans chaque puits, pendant 1h sous agitation à l'abri de la lumière. L'anticorps anti-claudine 1 (Invitrogen™) a été utilisé au 1/50^{ième}, la phalloïdine Alexa Fluor 488 (Molecular Probes® - Life Technologies™) au 1/50^{ième} et l'anticorps anti-filaggrine au 1/50^{ème} (Abcam®). Après plusieurs lavages avec du D-PBS, les cellules ont été mises à incuber 1h sous agitation à l'abri de la lumière avec l'anticorps secondaire au 1/2000^{ème}: Alexa Fluor 488 goat anti-rabbitIgG (H+L) (Molecular Probes® - Life Technologies™) ou Alexa Fluor®488 F(ab')2 anti mouse IgG (H+L) (Molecular Probes® - Life Technologies™) selon l'anticorps primaire. Plusieurs lavages avec du D-PBS ont été réalisés, suivis d'un lavage à l'eau distillée afin d'éliminer les sels restants. Les lames ont été montées avec du Prolong® Gold antifadereagent + DAPI (Molecular Probes® - Invitrogen™) et ont été observées à l'aide d'un microscope à fluorescence (Nikon Eclipse 50i).

### 1.1.4. Caractérisation du modèle par analyse génique

L'extraction d'ARN totaux a été automatisée par le QIACube (Qiagen) selon le protocole du kit RNeasy Mini Kit (Qiagen).

Les ARN totaux ont été dosés au NanoDrop® (Labtech® - ThermoScience).

La RT-PCR a été faite à partir des ARN totaux préalablement extraits. Les ARN messagers (ARNm) ont été rétro-transcrits en ADN complémentaires (ADNc) grâce à l'appareil Thermal Cycler® (AppliedBiosystems) avec le kit :ReadyScript™cDNASynthesis Mix(Sigma - Aldrich).

La PCR a été effectuée sur l'appareil ViiA™ 7 Real-Time PCR System (Applied Biosystems par Life Technologies™).

Le mix de PCR utilisé est TaqMan®Fast Advanced Master Mix 2X (AppliedBiosystems). Les résultats ont été analysés grâce aux logiciels ViiA 7 RUO Software et Expression Suite Software v1.0.

### 1.2. Résultats et Discussion

### 1.2.1. Modèle de cellules fragilisées

Le double stress combine deux simples stress ; celui d'altération au SDS et celui de la déshydratation à 24 h d'intervalle. Le but de ce double stress est de créer une fragilisation cellulaire par le premier stress. Si les cellules sont fragilisées, la réponse cellulaire au 2^{ème} stress devrait être exacerbée par rapport à des cellules qui n'ont pas subi de 1^{er} stress.

Deux cas différents ont été envisagés pour la caractérisation de ce modèle ; soit un premier stress au SDS (10 ou 15 µg/mL) suivi 24 h plus tard par un second stress de déshydratation (20 ou 25 min), soit inversement un premier stress de déshydratation (20 ou 25 min) suivi 24 h plus tard d'un second stress au SDS (10 ou 15 µg/mL). Les conditions montrées sont celles combinant un stress au SDS (10 µg/ml) suivi d'un stress de déshydratation (20 min).

### 1.2.2. Analyse protéique

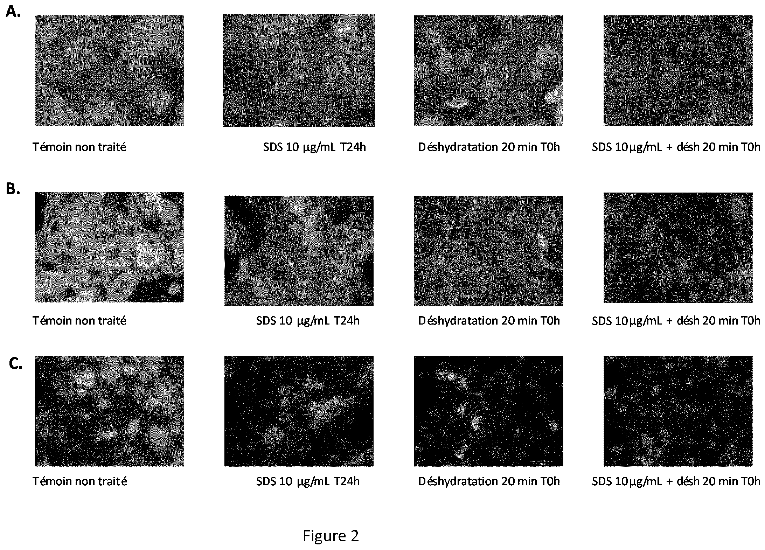

Les résultats à T0h du double stress (1) SDS, (2) déshydratation, sont montrés par rapport au témoin non traité et aux contrôles simples stress (SDS 10 µg/mL T24h et déshydratation 20 min T0h) équivalents à la condition de double stress (Figure 2).

Trois marquages ont été réalisés, la claudine 1 (protéine des jonctions serrées), la phalloïdine (cytosquelette d'actine) et la filaggrine (marqueur des kératinocytes différenciés participant à l'aggrégation des tonofilaments de kératine et à la formation du facteur naturel d'hydratation) (voir Figure 2).

Pour les trois marquages on observe qu'à T0h du double stress l'intensité de marquage est moindre et très hétérogène par rapport à celle du témoin, celle du simple stress SDS à T24h ou celle du simple stress de déshydratation à T0h. Ces résultats mettent en évidence une fragilisation des KNH par le premier stress au SDS 10 µg/mL et une altération de la fonction barrière au niveau protéique exacerbée par le cumul des deux stress.

### 1.2.3. Analyse de l'expression génique après un double stress :

### 1.2.3.1. Double stress « SDS puis déshydratation ».

L'étude de l'expression génique a été réalisée 4 h après le double stress « SDS puis déshydratation ».

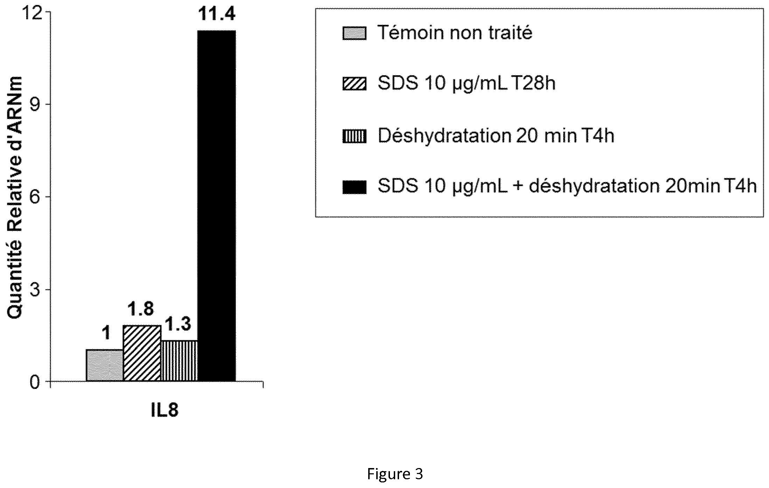

L'expression de la cytokine IL8 (Figure 3), produite par les cellules épithéliales en réponse à un stimulus inflammatoire, est à la limite de l'induction (QR=1,8) après 28 h du stress au SDS. IL8 n'est pas induit après 4 h d'un stress de déshydratation (QR=1,3). Cependant, après 4 h du double stress, IL8 est très fortement induit avec QR=11,4. Ces résultats permettent de dire qu'il y a une tendance à l'augmentation de l'inflammation 4 h après un double stress. Le cumul des deux stress exacerbe l'expression de l'IL8 dans les 4 h suivant le double stress. Dans cette expérience et dans le cas de l'IL8, on peut dire qu'il y a eu une fragilisation des cellules par le premier stress au SDS (cf. figure 3).

La caractérisation du modèle de « cellules fragilisées » a été établie pour un premier stress au SDS de 10 µg/mL suivi 24 h plus tard par un stress de déshydratation de 20 minutes. Le stress au SDS fragilise les cellules, l'état de la barrière physique est beaucoup plus altéré par le cumul des deux stress (cela même avec un intervalle de 24 h entre les deux) que par les simples stress. Après un double stress, les cellules montrent une désorganisation du cytosquelette d'actine, des jonctions serrées, une diminution du marquage de la filaggrine et une augmentation de l'inflammation (cytokine IL8). Ces altérations de la fonction barrière et cette inflammation sont amplifiées par la réalisation d'un premier stress 24 h avant le 2^{ème} stress. Le 1^{er} stress fragilise donc les cellules.

### 1.2.3.2. Double stress « déshydratation puis SDS ».

L'étude de l'expression génique a été réalisée 4 h après le double stress « déshydratation puis SDS ».

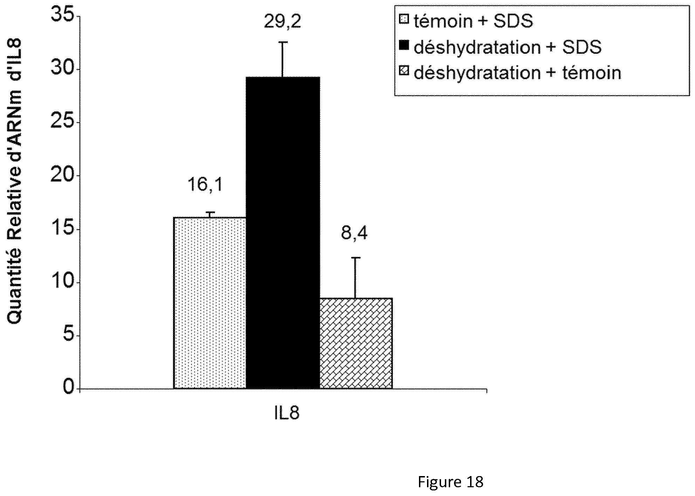

La quantité relative d'ARNm a été évaluée 4h après le deuxième stress (SDS). Ces conditions de double stress induisent de façon plus importante l'expression de certains gènes. Ainsi, l'expression des ARNm d'IL8 est induite de façon très reproductible avec un facteur d'induction supérieur à 1,8 par rapport à la condition « témoin + SDS » (figure 18). Cette induction n'est pas due au stress de déshydratation seul (« déshydratation + témoin ») : les quantités relatives d'ARNm y sont les plus faibles.

De façon générale, cette modulation montre que le premier stress de déshydratation a « fragilisé » les cellules. Le deuxième stress a induit une réponse plus importante quand les cellules ont été « fragilisées » par un premier stress que lorsqu'il n'y en a pas eu.

### Exemple 2 : Caractérisation du modèle tissulaire représentatif de peaux fragilisées (Figure 1B)

### 2.1. Matériels et Méthodes

### 2.1.1. Matériel Biologique

Cette étude a été réalisée sur des épidermes reconstruits humains selon la technique décrite par Poumay *(Poumay, Y., 2007).*

L'élaboration du modèle est réalisée sur des épidermes humains reconstruits fournis par le service d'Ingénierie Tissulaire Cutanée de Pierre Fabre. Ces derniers mesurent 0.63 cm² et sont réceptionnés (généralement par série de 48 épidermes) dans un milieu permettant leur croissance. Lors de la réception, les épidermes sont transférés dans des nouvelles plaques de 12 puits contenant du milieu DMEM *(Dulbecco'sModifiedEagle's Medium)* supplémenté en insuline, en transferrine et en sélénium, dans le but de les maintenir en survie. Afin de ne pas fausser les tests et dosages, ce milieu est dépourvu de rouge de phénol. Lors des incubations, les tissus reposent toujours dans du milieu et sont placés dans une étuve à 37°C contenant 5% de CO₂.

### 2.1.2. Développement d'un modèle de peau fragilisée

### 2.1.2.1. Stress au SDS :

Différentes concentrations de SDS ont été appliquées à la surface des épidermes pendant 18h et 4h. En s'appuyant sur une précédente étude, les concentrations suivantes ont été choisies : 0.4%, 0.2%, 0.1%, 0.15% et 0.05%. Cette gamme a permis de définir la concentration qui engendre une toxicité modérée et une légère altération de la barrière épidermique. En parallèle, les épidermes non traités ont reçu du tampon phosphate salin (PBS). Tous les épidermes ont été déposés dans l'incubateur pendant 18h ou 4h.

Les conditions retenues pour la suite des expériences sont : une concentration de SDS comprise entre 0,1% et 0.15%, pour un temps d'incubation (temps de récupération) de 2H à 24H.Il s'agit de conditions qui n'entraînent pas la mortalité cellulaire tout en altérant la fonction barrière et donc en fragilisant les cellules. Il s'agit donc des conditions maximales non cytotoxiques.

### 2.1.2.2. Stress de déshydratation :

La déshydratation des épidermes a été effectuée dans une enceinte climatique maintenue à 37°C. Pour mettre au point ce deuxième traitement, l'hygrométrie de l'enceinte climatique et le temps de déshydratation sont évalués. Ainsi, les tissus sont déshydratés pendant 1h, 1h30 et 2h à 15% et 30% d'humidité. Les tissus sont ensuite retransférés dans une étuve à 37°C contenant 5% de CO₂ pendant 4h ou 18h.

Les conditions retenues pour le stress de déshydratation dans la suite des expériences sont: un stress hydrique à 15% d'humidité, pour un temps d'incubation compris entre 1h et 1h20. Il s'agit de conditions qui n'entraînent pas la mortalité cellulaire tout en altérant la fonction barrière et donc en fragilisant les cellules.

Arrêt des traitements : l'incubation à partir de la fin du deuxième stress varie entre 2h et 48h.

### 2.1.2.3. Double-Stress

Les conditions retenues pour le double-stress sont : un premier stress au SDS 0,1% sur un temps d'incubation de 18h, puis, quelques heures avant la fin de l'incubation (4h) les épidermes sont rincés en surface avec du PBS avant l'application de formulations d'actifs, puis un stress de déshydratation est appliqué (15% d'humidité, pour un temps d'incubation de 1h). A la fin du deuxième stress, les épidermes sont replacés dans l'incubateur pendant 18h.

### 2.1.3. Paramètres analysés

Pour le stress par déshydratation et le Double Stress (SDS+ déshydratation), l'analyse a été faite 18 h après la fin du stress de déshydratation. Pour le stress au SDS, l'analyse a été faite :
=>soit 18 h après le début du stress (ce qui permet de démontrer une altération de la Fonction Barrière (chute de la TEER, induction de IL1alpha et réduction du marquage filaggrine)
=>soit 36h après, pour la comparaison avec le double stress.

### 2.1.3.1. Dosage de l'activité de la lactate déshydrogénase LDH

La fragilisation engendrée par ces différents stressa été mesurée par le dosage colorimétrique de la lactate déshydrogénase (LDH), une enzyme présente dans le cytoplasme des cellules. En cas de mort cellulaire, celle-ci est libérée dans le milieu après la rupture des membranes cellulaires.

### 2.1.3.2. Analyse histologique des tissus

Après les traitements, les épidermes ont été récupérés et inclus en paraffine afin de réaliser des coupes histologiques de 7 µm. La coloration Hemalun Eosine de ces coupes a permis d'observer la structure des différentes couches épidermiques au microscope optique.

### 2.1.3.3. Dosage des cytokines sécrétées

La réponse inflammatoire engendrée par ces différents stress a été déterminée par la quantification de deux cytokines : l'interleukine 1 alpha (IL1-α) et l'interleukine 8 (IL8). Ces deux protéines sont sécrétées par les kératinocytes comme premières lignes de défense aux différents stimuli extérieurs.

L'interleukine-1, responsable de la production de l'inflammation, a été dosée par une méthode immuno-enzymatique (ELISA).

L'interleukine IL8 est fortement impliquée dans l'amplification de la réponse inflammatoire. Ellea été dosée par une méthode d'Immunofluorescence : HTRF (de l'anglais : *homogeneous time resolved fluorescence*).

### 2.1.3.4. Caractérisation du modèle par analyse génique

L'extraction d'ARN totaux a été automatisée par le QIACube (Qiagen) selon le protocole du kit RNeasy Mini Kit (Qiagen).

Les ARN totaux ont été dosés au NanoDrop® (Labtech® - ThermoScience).

La RT-PCR a été faite à partir des ARN totaux préalablement extraits. Les ARN messagers (ARNm) ont été rétro-transcrits en ADN complémentaires (ADNc) grâce à l'appareil

Thermal Cycler® (AppliedBiosystems) avec le kit :ReadyScript™cDNASynthesis Mix(Sigma - Aldrich).

La PCR a été effectuée sur l'appareil ViiA™ 7 Real-Time PCR System (Applied Biosystems par Life Technologies™). Le mix de PCR utilisé est TaqMan®Fast Advanced Master Mix 2X (AppliedBiosystems). Les résultats ont été analysés grâce aux logiciels ViiA 7 RUO Software et Expression Suite Software v1.0.

### 2.1.3.5. Mesure de la résistance électrique trans-épithéliale

L'intégrité des jonctions serrées a été évaluée par la mesure de la résistance électrique trans-épithéliale (TEER). La résistance électrique résulte de la capacité des tissus cellulaires à former une barrière plus ou moins perméable. Elle reflète le passage des ions, des solutés et de l'eau à travers le tissu. Dans un épithélium, il existe deux voies de passage entre le compartiment apical et basal : le flux transcellulaire et paracellulaire. La mesure de la TEER se traduit en grande partie par le flux paracellulaire. La résistance de cette voie dépend de la résistance des jonctions serrées *(Rtj),* et de celle de l'espace intercellulaire *(Rics).* Ainsi, l'utilisation d'un Ohmmètre *(Millicel lERS*®*, Millipore)* permet la détermination de la TEER et l'estimation de l'intégrité des jonctions serrées.

### 2.1.3.6. Immunomarquage de la Filaggrine et de la claudine 1

Après le double stress, les épidermes ont été inclus dans l'O.C.T. et rapidement congelés dans une solution d'isopentane refroidie à -60°C par de l'azote liquide Les blocs peuvent être conservés à-80°C. Des coupes de 7 µmont ensuite été réalisées à l'aide d'un cryostat. Après avoir été fixés au méthanol à -20°C, les tissus ont été marqués et révélés suivant les instructions du kit *LSAB*+ *System Phosphatase alcaline FUCHSIN de DAKO*®

### 2.2. Résultats: double-stress [SDS + déshydratation]

### 2.2.1. Dosage de l'activité de la Lactate déshydrogénase LDH

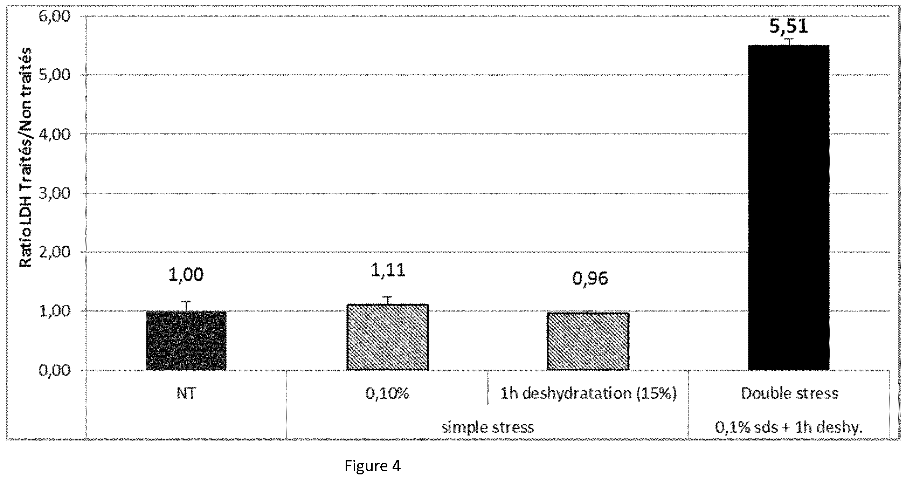

La fragilité engendrée par les simples stress n'est pas observable avec la mesure de la LDH. En revanche, elle est notable avec le double stress puisque le ratio augmente autour d'une valeur x5 (cf. figure 4). Ceci suggère qu'un degré de fragilisation a été atteint avec le double stress par un effet synergique de l'association des simples stress.

### 2.2.2. Analyse histologique des tissus

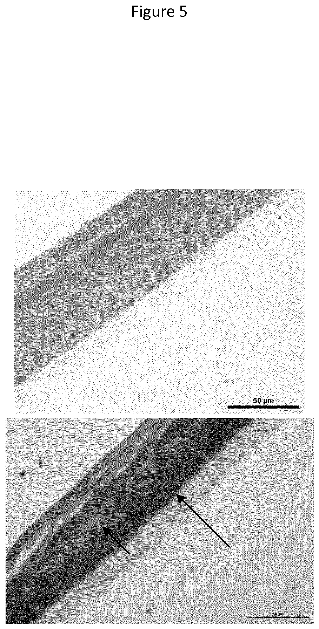

L'observation des coupes histologiques a révélé que le stress SDS ainsi que le double stress engendrent des altérations morphologiques modérées (cf. figure 5, voir les flèches): disparition de l'aspect granuleux de la couche granuleuse, présence de cellules plus grosses dans les couches suprabasales mais pas de présence visible de noyaux condensés dans la couche basale. Il n'y a pas de perte de viabilité du tissu (cf. figure 5).

### 2.2.3. Dosage des cytokines sécrétées

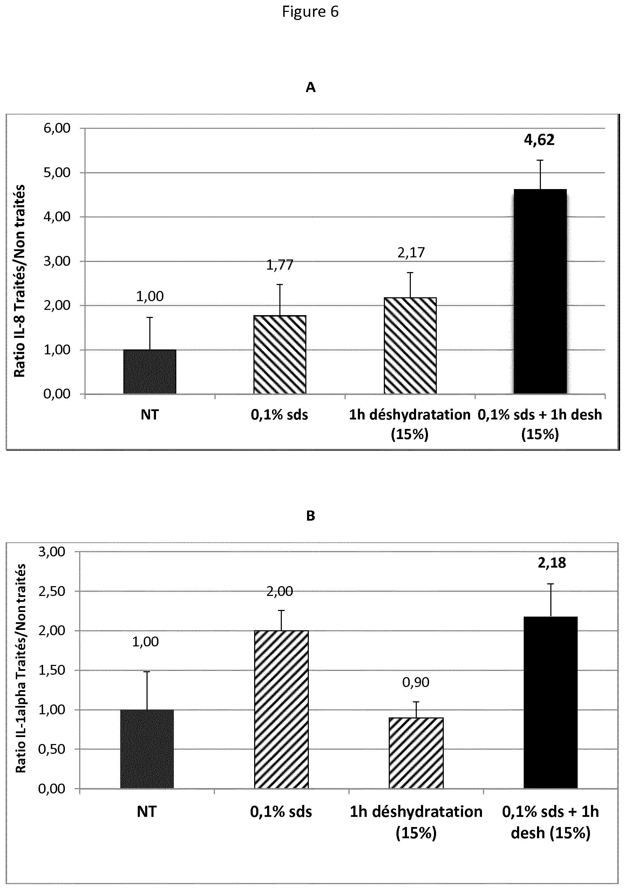

Le simple stress SDS induit une légère augmentation de la production d'IL1α et d'IL8. Le simple stress déshydratation induit une augmentation de la production d'IL1α. Dans le double stress on observe une potentialisation de la production d'IL8 (cf. figures 6A et 6B).

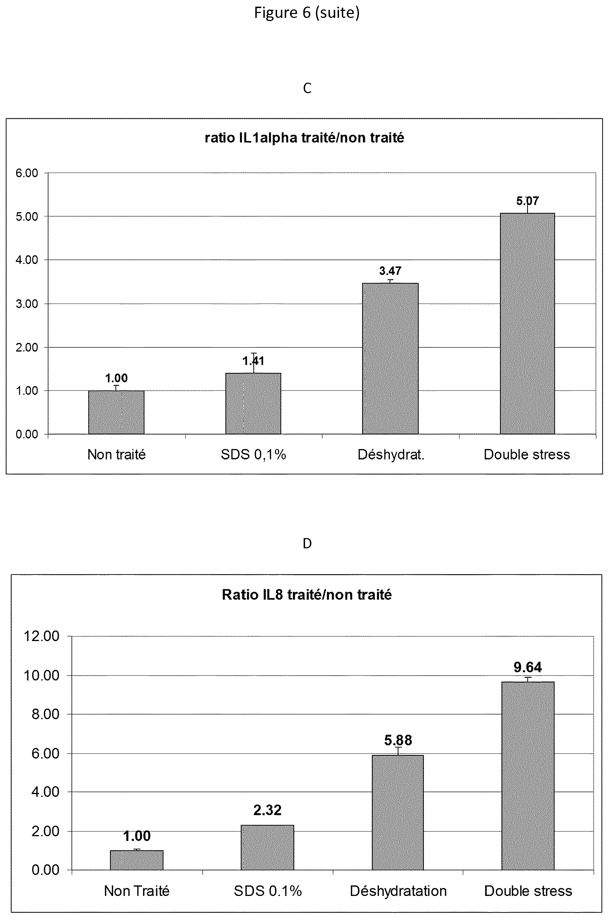

Dans une autre série d'expérience, la réponse inflammatoire du double stress a été comparée à celle observée dans une situation de simple stress, mais à 36h d'incubation post-stress SDS (cf. figures 6C et 6D).

Dans cette expérience, la production d'IL1α, qui avait été induite 18h après le simple stress SDS dans les expériences précédentes, est revenu à l'état basal 36h après ce stress (figure 6C). Le double stress induit quant à lui une nette augmentation d'IL1α, supérieure à l'induction de l'IL1α due au stress déshydratation seul. Il y a donc amplification de la réponse inflammatoire dans le double stress.

Pour l'IL8 (figure 6D), on retrouve aussi une nette amplification de la réponse inflammatoire dans le double stress.

### 2.2.4. Analyse de l'expression génique après un double stress

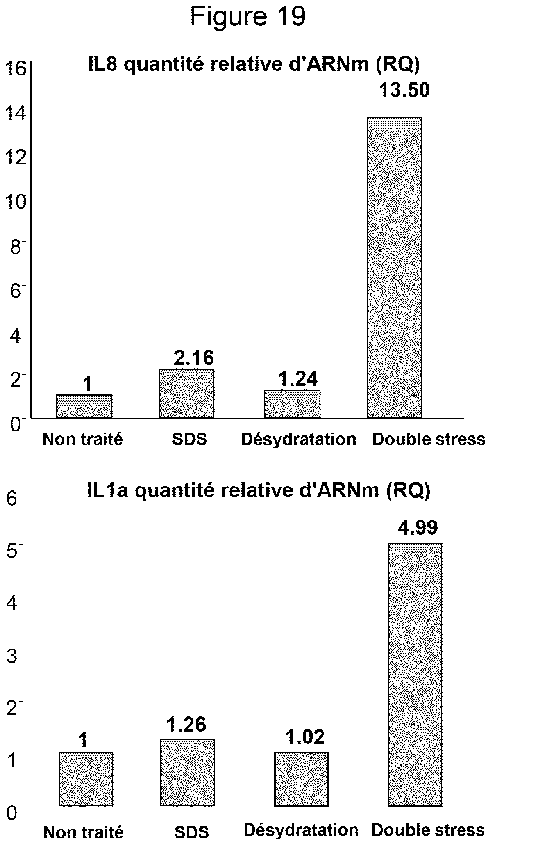

De la même manière que l'analyse de la production des cytokines IL1 alpha et IL8, on retrouve une nette exacerbation de l'induction des gènes codant pour IL1 alpha et IL8 (cf. Figure 19).

### 2.2.5. Mesure de la TEER

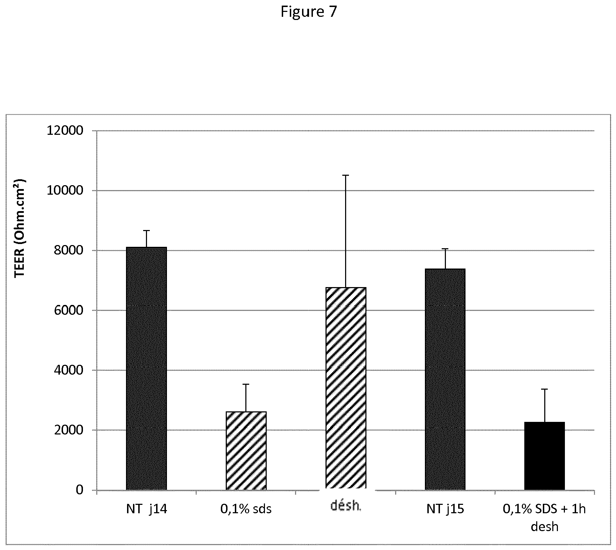

La mesure de la TEER à la fin du simple stress SDS témoigne que le SDS a bien fragilisée la fonction barrière de l'épiderme (cf. figure 7).

Le stress déshydratation ne semble pas avoir un effet sur la TEER. On observe à l'issue du double stress une diminution plus accentuée de la TEER par rapport à la mesure effectuée après le simple stress au SDS. Ceci est en accord avec la fragilisation accentuée de l'épiderme par le double stress.

### 2.2.6. Immunomarquage de la filaggrine

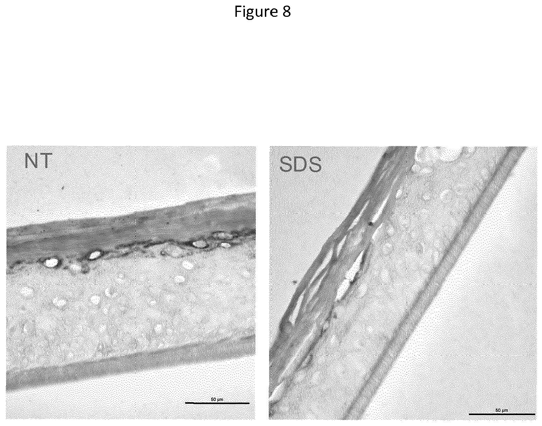

Le stress au SDS entraine une perte du marquage de la filaggrine(cf. figure 8). Ceci démontre que le stress SDS a bien altéré la fonction barrière de l'épiderme.

### CONCLUSION :

En conclusion, le 1^{er} stress appliqué sur le modèle tissulaire permet d'obtenir une peau fragilisée *in vitro.* En effet, ce stress entraine une altération de la fonction barrière observable et mesurable qui se caractérise par :
- une chute de la résistance électrique transépithéliale (TEER) démontrant une fragilisation de la fonction barrière,
- une altération ultra structurale avec disparition de l'aspect caractéristique de la couche granuleuse,
- une altération du marquage de la filaggrine,
- une induction modérée d'une inflammation cutanée (IL1α et IL8).

Par ailleurs, le deuxième stress permet de soumettre la peau fragilisée à un stress environnemental. Ce stress seul entraine une induction modérée d'une inflammation cutanée (IL1alpha et IL8).

Le double stress révèle une aggravation du phénotype observable avec le premier stress (SDS) qui se caractérise par :
- une chute accentuée de la TEER traduisant une altération plus importante de la barrière,
- une augmentation de la cytotoxicité induite
- une augmentation de la production de cytokines inflammatoires telles que l'IL8,
- une aggravation ultrastructurale avec une diminution du marquage de la filaggrine et de la claudine-1.

Ainsi ce double stress met en évidence une réponse amplifiée de la peau fragile lorsqu'elle est soumise à une agression environnementale.

### Exemple 3 :

### 3.1. Matériels et Méthodes

### 3.1.1. Matériel Biologique

La culture cellulaire a été réalisée à partir de kératinocytes normaux humains (KNH) issus d'explants de peau (abdominoplastie ou diminution mammaire). Les KNH ont été cultivés en condition stérile avec du milieu Keratinocyte-Serum Free Medium (K-SFM) (Gibco® - Life Technologies™) complémenté avec du Bovine PituitaryExtract (BPE) et de l'EpidermalGrowth Factor Human recombinant (EGF) (Gibco®- Life Technologies™). Le milieu a été complémenté en antibiotique par de la Primocin™ (InvivoGen). Les cellules ont été cultivées dans un incubateur à 37 °C sous atmosphère saturée en eau à 5 % de CO₂.

### 3.1.2. Modèle de cellules fragilisées : double stress SDS puis UV

Le premier stress avec SDS a été réalisé par une incubation des cellules 1 heure à 37 °C à la concentration souhaitée de SDS (Sigma-Aldrich). A la suite du stress, les cellules ont été rincées au Dulbecco's Phosphate Buffered Saline (1X) [-] Cacl₂ [-] MgCl₂ (D-PBS) (Gibco®-Life Technologies™) et remises en culture au temps T₀ avec du K-SFM sans compléments. 24 heures après le premier stress (SDS) les cellules ont subi un second stress différent du premier (UV spectre total).

Les cellules ont été irradiées avec une dose de 1.65 J/cm² d'UV totaux soit 1,53 J/cm² d'UVA et 120 mJ/cm² d'UVB.

Après le stress UV, les cellules ont été remises en culture au temps T₀ avec du DMEM sans SVF.

### 3.1.3. Caractérisation du modèle par analyse protéique

1 heure après le 2^{ème} stress, les cellules ont été fixées avec une solution d'acétone à - 20 °C pendant 10 minutes. Les membranes cellulaires ont été perméabilisées avec du Triton® X-100 (Sigma-Aldrich) à 0,1 % pendant 15 minutes. Après plusieurs rinçages avec du D-PBS, les sites spécifiques ont été saturés avec de l'Albumine de Sérum Bovin (BSA) (Sigma® Life science) à 3 % pendant 15 minutes. Puis, la phalloïdine Alexa Fluor 488 (Molecular Probes® - Life Technologies™), utilisée au 1/80^{ième}, a été ajoutée dans chaque puits, pendant 1h sous agitation à l'abri de la lumière. Plusieurs lavages avec du D-PBS ont été réalisés, suivis d'un lavage à l'eau distillée afin d'éliminer les sels restants. Les lames ont été montées avec du Prolong® Gold antifadereagent + DAPI (Molecular Probes® - Invitrogen™) et ont été observées à l'aide d'un microscope à fluorescence (Nikon Eclipse 50i).

### 3.1.4. Caractérisation du modèle par analyse génique

L'extraction d'ARN totaux a été automatisée par le QIACube (Qiagen) selon le protocole du kit RNeasy Mini Kit (Qiagen).

Les ARN totaux ont été dosés au NanoDrop® (Labtech® - ThermoScience).

La RT-PCR a été faite à partir des ARN totaux préalablement extraits. Les ARN messagers (ARNm) ont été rétro-transcrits en ADN complémentaires (ADNc) grâce à l'appareil Thermal Cycler® (AppliedBiosystems) avec le kit: ReadyScript™cDNASynthesis Mix(Sigma - Aldrich).

La PCR a été effectuée sur l'appareil ViiA™ 7 Real-Time PCR System (Applied Biosystems par Life Technologies™).

Le mix de PCR utilisé est TaqMan®Fast Advanced Master Mix 2X (AppliedBiosystems). Les résultats ont été analysés grâce aux logiciels ViiA 7 RUO Software et Expression Suite Software v1.0.

### 3.2. Résultats et Discussion

### 3.2.1. Modèle de cellules fragilisées

Le double stress combine deux simples stress : celui d'altération au SDS et celui d'irradiation aux UV totaux à 24 h d'intervalle. Le but de ce double stress est de créer une fragilisation cellulaire par le premier stress. Si les cellules sont fragilisées, la réponse cellulaire au 2^{ème} stress devrait être exacerbée par rapport à des cellules qui n'ont pas subi de 1^{er} stress.

### 3.2.2. Analyse protéique

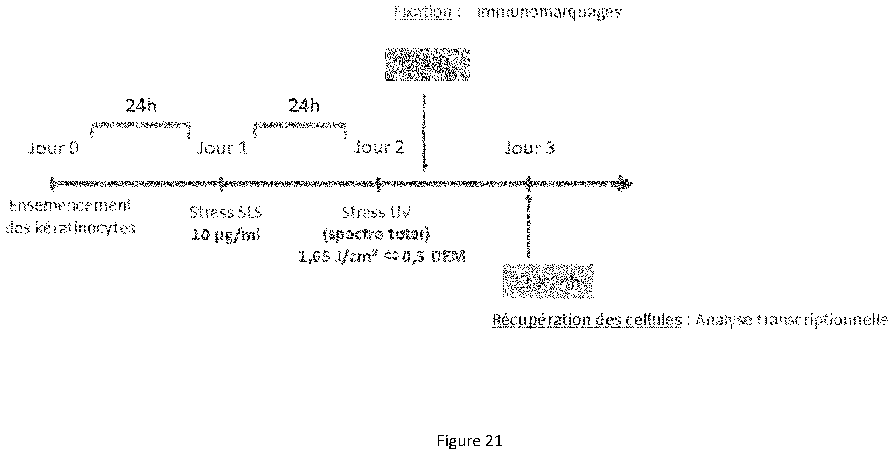

Les résultats à T1h du double stress (1) SDS, (2) UV totaux, sont montrés par rapport au témoin non traité et aux contrôles simples stress (SDS 10 µg/mL T25h et UV 1.65 J/cm² T1h) équivalents à la condition de double stress (Figure 21).

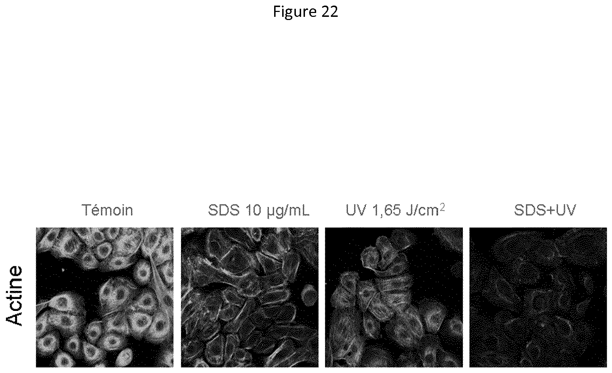

Le marquage à la phalloïdine (cytosquelette d'actine) a été réalisé (voir Figure 22).

On observe qu'à T1h du double stress l'intensité de marquage est très faible par rapport à celle du témoin, celle du simple stress SDS à T25h ou celle du simple stress de déshydratation à T1h. Ces résultats mettent en évidence une fragilisation des KNH par le premier stress au SDS 10 µg/mL et une altération de la fonction barrière exacerbée par le cumul des deux stress.

### 3.2.3. Analyse de l'expression génique après un double stress :

L'étude de l'expression génique a été réalisée 24 h après le double stress « SDS puis UV ». La quantité relative d'ARNm a été évaluée 24h après le deuxième stress (UV).

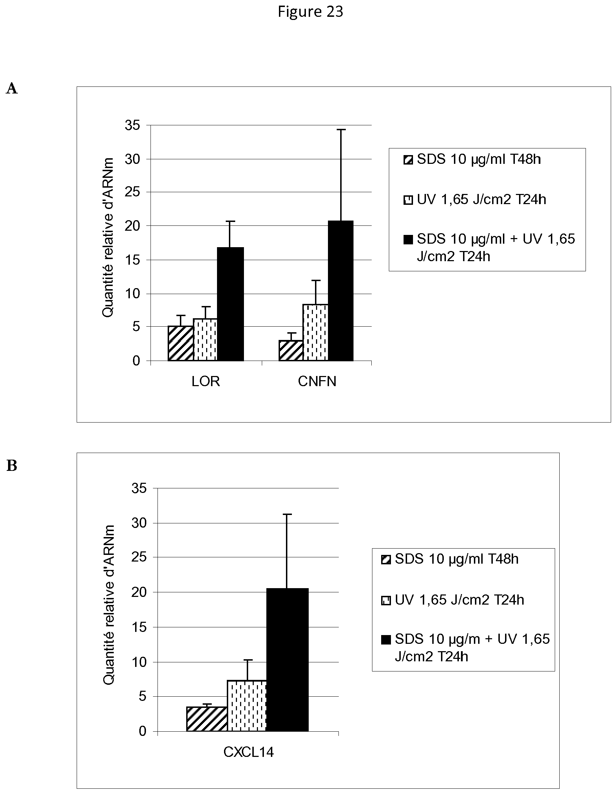

L'expression de deux marqueurs de la fonction barrière, la loricrine (LOR) et la cornifeline (CNFN), est induite après 48 h du stress au SDS (QR=5.1 et 3, respectivement) et après 24 h du stress aux UV totaux (QR=6.2 et 8.3, respectivement) (Figure 23A). Cependant, après 24 h du double stress, l'expression de la LOR et de la CNFN est encore plus fortement induite avec des QR=16.8 et 20.7 respectivement. Ces résultats permettent de dire qu'il y a une altération de la fonction barrière après ces stress et que le cumul des deux stress exacerbe l'expression de la LOR et de la CNFN dans les 24 h suivant le double stress.

De même l'expression de la chémokine CXCL14 (marqueur de l'inflammation et de l'immunité innée) est induite de façon plus importante par le double stress (QR=20.5) que parles simples stress SDS (QR=3.4) ou UV (QR=7.2) seuls (Figure 23B).

La caractérisation du modèle de « cellules fragilisées » a été établie pour un premier stress au SDS de 10 µg/mL suivi 24 h plus tard par une irradiation aux UV totaux. Le stress au SDS fragilise les cellules, l'état de la barrière physique est beaucoup plus altéré par le cumul des deux stress (cela même avec un intervalle de 24 h entre les deux) que par les simples stress. Après un double stress, les cellules montrent une désorganisation du cytosquelette d'actine, une altération de la fonction barrière (LOR et CNFN), de l'immunité innée et de l'inflammation (chémokine CXCL14). Ces altérations de la fonction barrière et cette inflammation sont amplifiées par la réalisation d'un premier stress 24 h avant le 2^{ème} stress. Le 1^{er} stress fragilise donc les cellules.

De façon générale, cette modulation montre que le premier stress a « fragilisé » les cellules. Le deuxième stress a induit une réponse plus importante quand les cellules ont été « fragilisées » par un premier stress que lorsqu'il n'y en a pas eu.

### Exemple 4 : Evaluation d'actifs suivant le modèle cellulaire

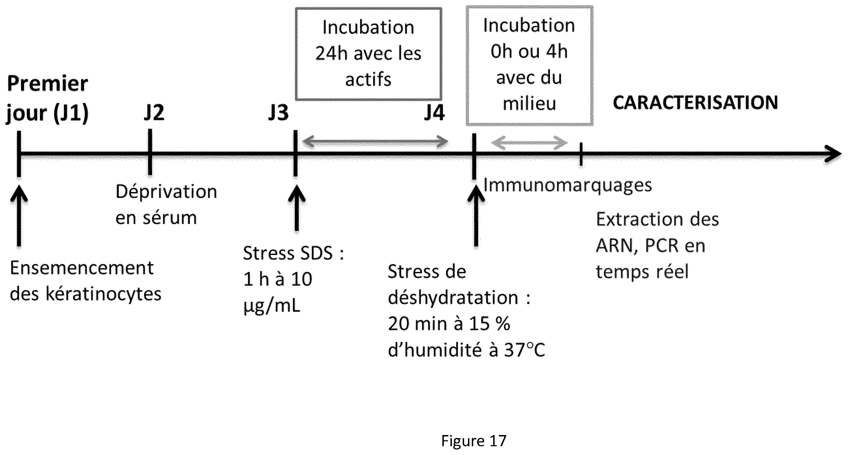

Un extrait de plantules d'Avoine nommé C a été testé sur ce modèle dans le but d'évaluer son action préventive contre le second stress sur les cellules fragilisées par un premier stress au SDS. Deux concentrations d'actif (10 et 100 µg/mL) et différents temps de récupération (immédiatement après le stress et 4 h après le double stress) ont été testés. Le mode opératoire de l'expérience est schématisé sur la figure 17.

### Préparation de l'extrait :

- Extraire 10 g de plantules d'avoine broyées avec 100 ml de solvant d'extraction acétone / eau (80/20) (v/v).
- Filtrer et rincer le marc avec le solvant d'extraction.
- Evaporer l'acétone et reprendre la phase aqueuse.
- Filtrer. Concentration jusqu'à obtenir environ 70% de matière sèche.

L'évaluation de l'actif a été faite par rapport à un contrôle positif. La molécule utilisée est le tréhalose (10 µM), elle protège les membranes cellulaires et les protéines de la déshydratation.

### 4.1. Analyse protéique

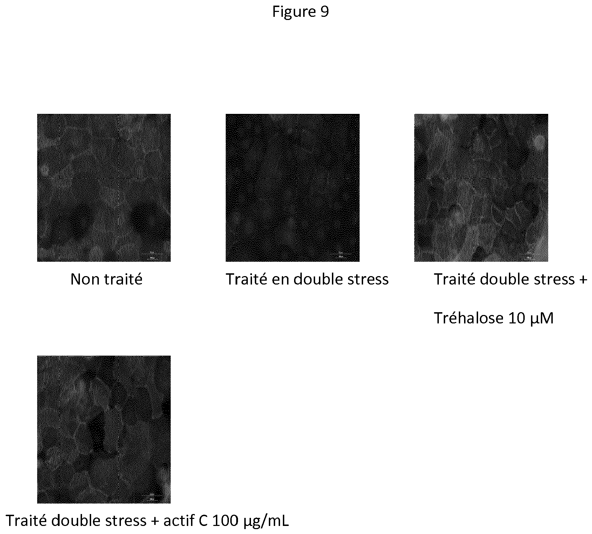

L'actif C a été testé à 100 µg/mL immédiatement après le double stress (SDS 10 µg/ml suivi de 20 min de déshydratation) à l'aide d'immunomarquages, le but étant de déterminer et comparer l'activité de l'actif et la concentration permettant une protection optimale contre le 2^{ème} stress de déshydratation. Trois marquages ont été réalisés : le marquage de la claudine 1, de l'actine (par la phalloïdine) et de la filaggrine (cf. figures 9, 10 et 11).

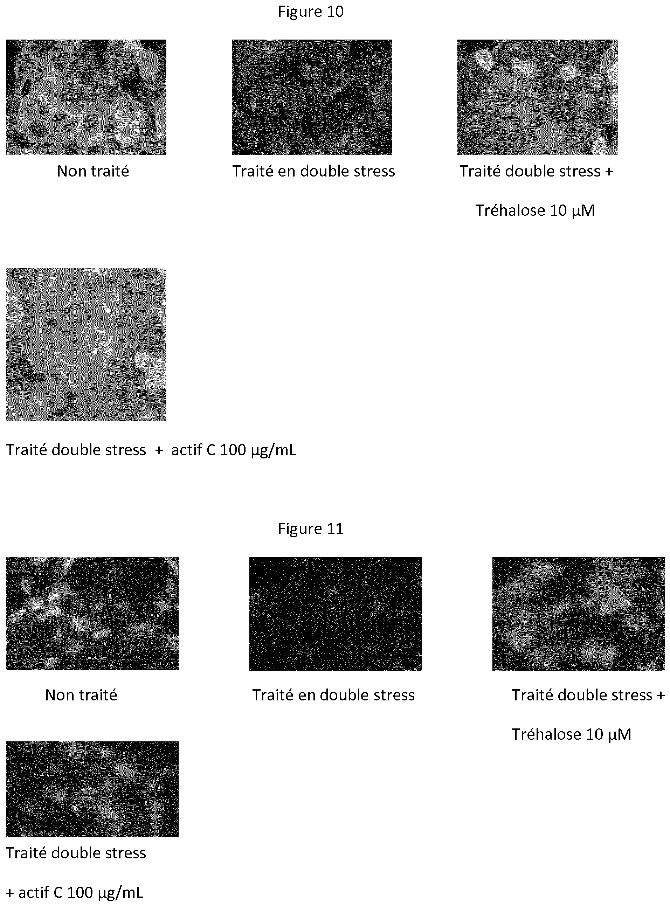

Le double stress entraîne immédiatement une forte altération des jonctions serrées. Ceci est visualisé par une perte de l'intensité et une hétérogénéité du marquage de la claudine 1 à T0h. Le contrôle positif, le tréhalose, montre un marquage proche de celui obtenu avec le témoin en termes d'intensité et d'homogénéité de la fluorescence. Il limite la désorganisation des jonctions serrées. L'actif C à 100 µg/mL permet une protection presque totale de l'organisation des jonctions serrées contre le second stress de déshydratation. Les marquages obtenus immédiatement après le double stress avec l'actif C, montrent un marquage homogène, bien localisé sur la membrane cellulaire et d'intensité similaire au témoin non traité. En comparant ces résultats à la condition double stress sans actif, on peut donc conclure que les cellules au contact de C ont été protégées du 2^{ème} stress de déshydratation (cf. figure 10).

Le double stress entraîne immédiatement une modification du réseau d'actine visualisée par une perte de l'intensité et une hétérogénéité du marquage. Le contrôle positif, le tréhalose, montre un marquage intense et homogène, proche du marquage de la condition témoin. Le réseau d'actine n'est pas désorganisé comme dans le cas de la condition double stress sans actif. Le tréhalose limite la désorganisation du cytosquelette d'actine.

Les traitements en présence de l'actif C à 100 µg/mL ont permis une bonne protection contre le stress de déshydratation. Les marquages sont très proches de celui obtenu dans la condition témoin non traité en termes d'intensité de fluorescence, d'homogénéité et de présence de filaments d'actine (cf. figure 11).

Immédiatement à la suite d'un double stress, le marquage de la filaggrine n'est presque plus présent au niveau des cellules par rapport au témoin non traité. En présence de tréhalose le marquage de la filaggrine est toujours présent, un peu moins que dans le témoin non traité et de façon un peu plus hétérogène mais avec une intensité identique.

En présence de l'actif C, le marquage de la filaggrine est toujours présent immédiatement après le double stress. Les marquages sont assez homogènes et l'intensité des zones marquées est similaire à celle du témoin. Cet actif à 100 µg/mLa permis une bonne protection du marquage de la filaggrine par rapport au deuxième stress de déshydratation (cf figure 9).

### 4.2. Analyse de l'expression génique sur un double stress en présence d'actifs

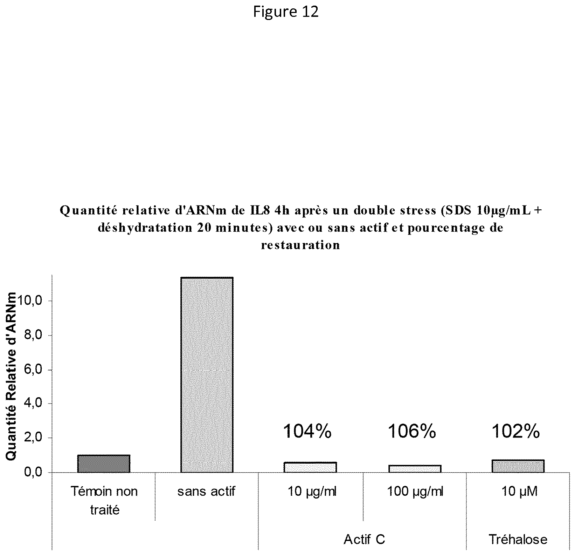

Dans les 2 expériences réalisées, l'IL8, marqueur de l'inflammation liée au stress, est modulé de façon reproductible et son expression est inhibée par la présence des actifs (Figure 12). A partir de ces résultats, un pourcentage de restauration de la quantité relative d'ARNm a été défini pour l'actif C et ce pour chaque concentration (10 et 100 µg/mL) 4 h après le second stress.

Dans cette expérience, l'IL8 est très fortement induite 4 h après le double stress (QR=11). Le contrôle positif montre une restauration totale de la quantité relative d'ARNm après 4 h (102 % de restauration). L'actif C, à 10 ou à 100 µg/mL, restaure aussi complètement l'expression de l'IL8 (restauration entre 97 à 106 %).

L'actif C étant mis en contact avec les cellules après le 1^{er} stress, ce modèle permet d'analyser son effet sur la protection vis à vis d'un 2^{ème} stress sur des cellules « fragilisées ». D'un point de vue général, au niveau protéique et à 100 µg/mL, cet actif permet une protection de la désorganisation de la claudine 1, du réseau d'actine et du marquage de la filaggrine contre le stress de déshydratation. De plus, il restaure complètement l'expression des ARNm de l'IL8.

### Exemple 5 : Evaluation de principes actifs sur modèle tissulaire

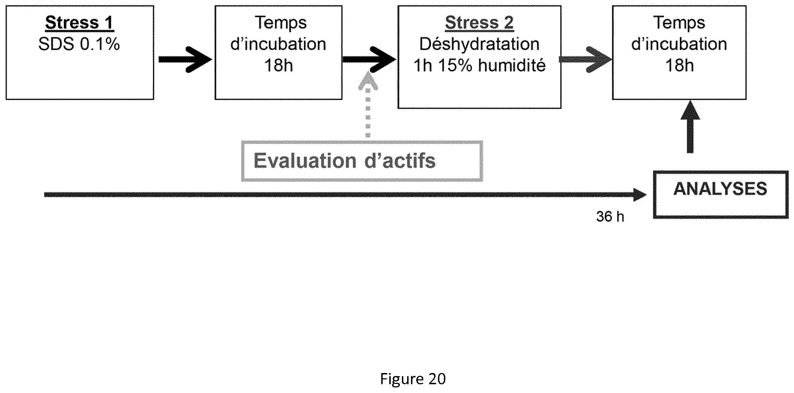

Dans cette étude, le même actif C à base de plantules d'Avoine (cf. exemple 3) a été testé afin d'étudier son effet sur la réponse au deuxième stress. Le mode opératoire de l'expérience est schématisé sur la figure 20.

L'extrait végétal, pré-formulé à différentes concentrations, a été appliqué à la surface des tissus à raison de 5,5 mg/cm² sur des épidermes reconstruits entre les deux stress. La formulation est une base minimale aqueuse. Les formules ont été appliquées après le premier stress au SDS (épidermes « fragilisés») et quelques heures avant le deuxième stress hydrique.

Dans cette étude, un extrait de plantules d'avoine a été évalué à différentes concentrations (3% et 5%).

### 5.1. Etude de la cytotoxicité

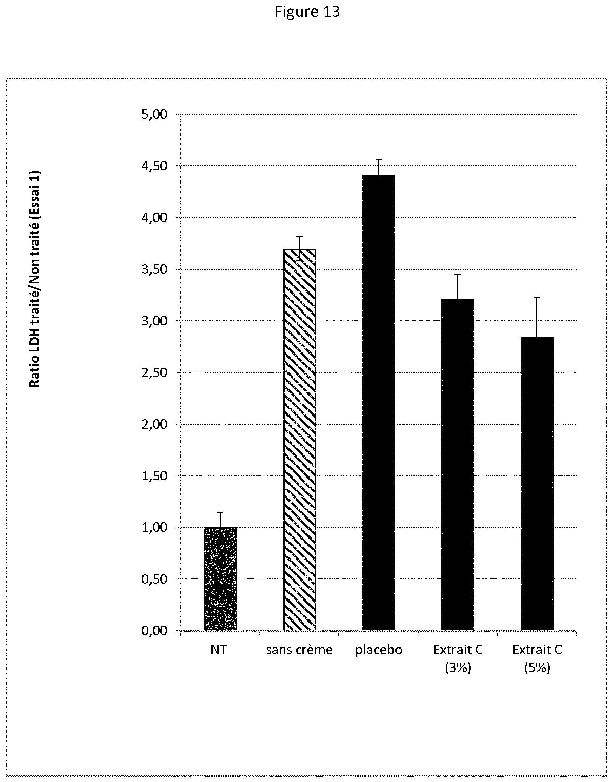

L'ajout de l'extrait C (3% et 5%) dans la formule a permis un net effet protecteur. Effectivement, leur ratio LDH a été diminué par rapport au ratio des épidermes témoins (double stress sans crème) et nettement plus faible que celui de la condition formule placebo (cf. figure 13).

### 5.2. Etude de la réponse inflammatoire : IL-lalpha

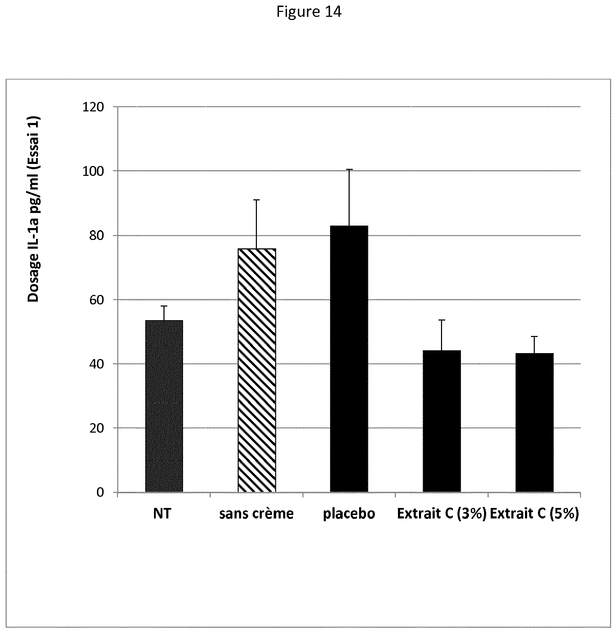

La production d'IL-1α a été induite dans les surnageants des épidermes traités par le double stress. L'application des formulations contenant l'extrait C à 3 et 5% a limité fortement la libération d'IL-1α. En effet, en présence de l'extrait C, le taux d'IL-1α est identique à celui des épidermes non traités (cf. figure 14). Ceci suggère que l'extrait C a une propriété anti-inflammatoire.

### 5.3.Immunomarquagefilaggrine et claudine-1

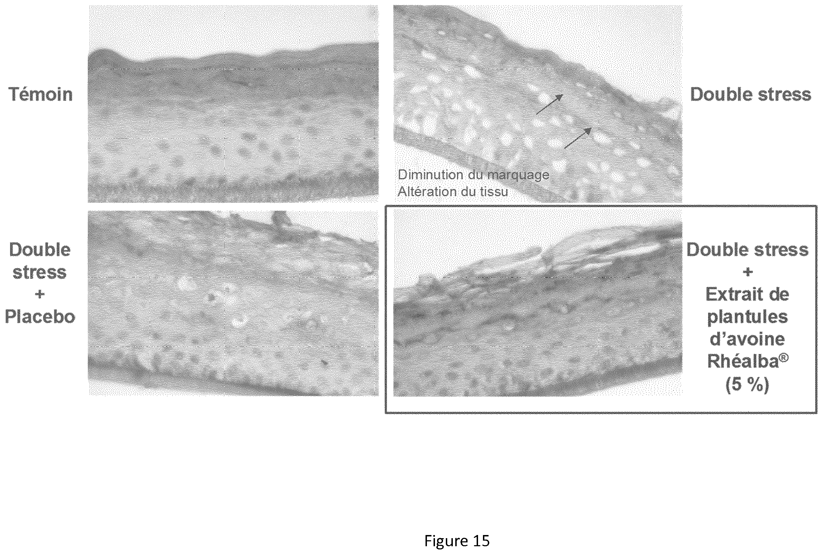

L'actif C préserve et restaure le marquage de la filaggrine qui avait été drastiquement diminué par le double-stress (cf. figure 15).

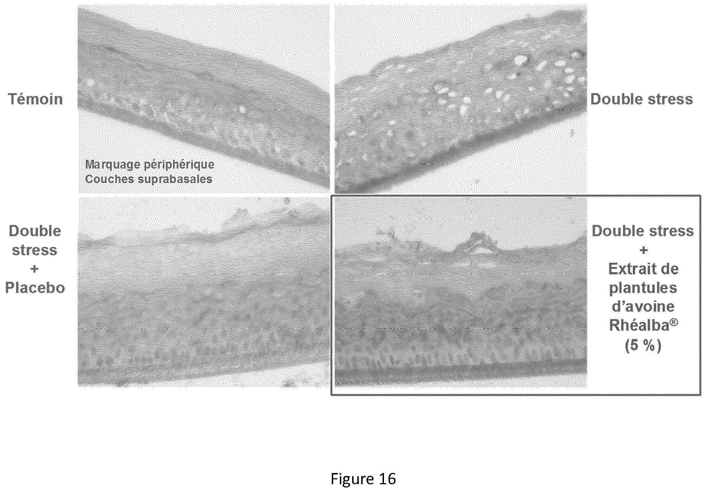

De la même manière, l'actif C a préservé et restauré le marquage de la claudine-1, attestant de la protection/restauration des jonctions serrées et de la fonction barrière (cf. figure 16).

### CONCLUSION

L'actif C incorporé dans une formule simple montre des propriétés de protection intéressantes vis à vis du 2^{ème} stress effectué après un 1^{er} stress.

L'étude réalisée sur les épidermes reconstruits valide donc les résultats obtenus sur le modèle cellulaire.

### Références bibliographiques

Biniek K. et al "Solar UV radiation reduces the barrier function of human skin", PNAS 2012, 109(42):17111-6
Haftek M. et al., "Epidemiology of "fragile skin": results from a survey of different skin types". ClinCosmetInvestigDermatol.2013;Dec 2;6:289-94
Iordanov M.S. et al, "Ultraviolet radiation triggers the ribotoxic stress response in mammalian cells". J. Biol. Chem. 1998; 273:15794-15803
Limat et al., "Use of epidermal equivalents generated from follicular outer root sheath cells in vitro and for autologous grafting of chronic wounds". Cells Tissues Organs 2002; 172(2):79-85
Poumay Y. et al., "A simple reconstructed human epidermis: preparation of the culture model and utilization in in vitro studies." Arch Dermatol Res. 2004;Oct;296(5):203-11.
Poumay Y.et al. 'Modelling the human epidermis in vitro: tools for basic and applied research." Arch Dermatol Res.2007Jan;298(8):361-9.
Ramos e Silva M. et al, « Effects of age (neonates and elderly) on skin barrier function » Exp. Dermatol., 2013. 22(5):329-35
Schiraldi C. et al, "Trehalose production: exploiting novel approaches" Trends Biotechnol.2002;Oct;20(10):420-5

## Revendications

1. Procédé *in vitro* ou *ex vivo* de fragilisation de cellules de l'épiderme, contenant au moins deux traitements choisis parmi : un traitement chimique, un traitement déshydratant réalisé en plaçant lesdites cellules dans un compartiment contenant une humidité comprise entre 5 et 40% et ayant une température comprise entre 30 et 40°C, un traitement physique et un traitement mécanique, **caractérisé en ce que** ledit traitement chimique est réalisé en mettant en contact lesdites cellules avec des détergents ou des cétones,**en ce que** ledit traitement physique est une irradiation UV et **en ce que** ledit traitement mécanique est une compression ou une traction.

2. Procédé selon la revendication 1, **caractérisé en ce que** ledit traitement chimique est réalisé en mettant en contact lesdites cellules avec du Sodium Dodécyl Sulfate (SDS).

3. Procédé selon l'une quelconque des revendications 1 à 2, **caractérisé en ce que** lesdites cellules sont isolées.

4. Procédé selon la revendication 3, **caractérisé en ce que** ledit traitement chimique est maintenu pendant une durée comprise entre 30 minutes et 4 heures, de préférence 1 heure.

5. Procédé selon la revendication 3 ou 4, **caractérisé en ce que** ledit traitement déshydratant est maintenu pendant une durée comprise entre 10 minutes et 40 minutes, de préférence entre 20 et 25 minutes.

6. Procédé selon l'une quelconque des revendications 1 à 2, **caractérisé en ce que** lesdites cellules sont contenues dans un tissu, de préférence dans un épiderme reconstruit.

7. Procédé selon la revendication 6, **caractérisé en ce que** ledit traitement chimique est maintenu pendant une durée comprise entre 2 heures et 24 heures, de préférence 18 heures.

8. Procédé selon la revendication 6 ou 7, **caractérisé en ce que** ledit traitement déshydratant est maintenu pendant une durée comprise entre 30 minutes et 2 heures, de préférence 1 heure.

9. Procédé selon l'une quelconque des revendications 1 à 8, **caractérisé en ce que** les cellules de l'épiderme subissent un traitement chimique et un traitement déshydratant.

10. Procédé selon l'une quelconque des revendications 1 à 9, **caractérisé en ce que** lesdits traitements sont espacés de 4h à 30h.

11. Méthode de criblage pour l'identification *in vitro* de composés destinés au soin ou à la protection des peaux fragiles, comprenant la mise en contact desdits composés avec un modèle cellulaire ou tissulaire de peau fragilisée obtenu par le procédé selon l'une des revendications 1 à 10, **caractérisée en ce qu'**un composé candidat sera sélectionné si, en sa présence, on observe une restauration de la fonction barrière.

## Patentansprüche

1. *In vitro-* oder ex *vivo*-Verfahren der Versprödung von Zellen der Epidermis, beinhaltend mindestens zwei Behandlungen, gewählt aus: einer chemischen Behandlung, einer Dehydrierungsbehandlung, die durchgeführt wird indem die Zellen in ein Kompartiment, beinhaltend eine Luftfeuchtigkeit im Bereich zwischen 5 und 40 % und eine Temperatur im Bereich zwischen 30 und 40 °C aufweisend, platziert werden, einer physikalischen Behandlung und einer mechanischen Behandlung, **dadurch gekennzeichnet, dass** die chemische Behandlung durch In-Kontakt-Bringen der Zellen mit Detergenzien oder Ketonen durchgeführt wird, dadurch, dass die physikalische Behandlung eine UV-Bestrahlung ist und dadurch, dass die mechanische Behandlung eine Komprimierung oder eine Zugbeanspruchung ist.

2. Verfahren nach Anspruch 1, **dadurch gekennzeichnet, dass** die chemische Behandlung durch In-Kontakt-Bringen der Zellen mit Natriumdodecylsulfat (SDS) durchgeführt wird.

3. Verfahren nach einem der Ansprüche 1 bis 2, **dadurch gekennzeichnet, dass** die Zellen isoliert sind.

4. Verfahren nach Anspruch 3, **dadurch gekennzeichnet, dass** die chemische Behandlung während einer Dauer im Bereich zwischen 30 Minuten und 4 Stunden, vorzugsweise 1 Stunde, aufrecht erhalten wird.

5. Verfahren nach Anspruch 3 oder 4, **dadurch gekennzeichnet, dass** die Dehydrierungsbehandlung während einer Dauer im Bereich zwischen 10 Minuten und 40 Minuten, vorzugsweise zwischen 20 und 25 Minuten, aufrecht erhalten wird.

6. Verfahren nach einem der Ansprüche 1 bis 2, **dadurch gekennzeichnet, dass** die Zellen in einem Gewebe enthalten sind, vorzugsweise in einer rekonstruierten Epidermis.

7. Verfahren nach Anspruch 6, **dadurch gekennzeichnet, dass** die chemische Behandlung während einer Dauer im Bereich zwischen 2 Stunden und 24 Stunden, vorzugsweise 18 Stunden, aufrecht erhalten wird.

8. Verfahren nach Anspruch 6 oder 7, **dadurch gekennzeichnet, dass** die Dehydrierungsbehandlung während einer Dauer im Bereich zwischen 30 Minuten und 2 Stunden, vorzugsweise 1 Stunde, aufrecht erhalten wird.

9. Verfahren nach einem der Ansprüche 1 bis 8, **dadurch gekennzeichnet, dass** die Zellen der Epidermis einer chemischen Behandlung und einer Dehydrierungsbehandlung unterzogen werden.

10. Verfahren nach einem der Ansprüche 1 bis 9, **dadurch gekennzeichnet, dass** die Behandlungen von 4 h bis 30 h beabstandet sind.

11. Screening-Verfahren zur *in vitro-*Identifikation von Verbindungen, die zur Pflege oder zum Schutz spröder Häute bestimmt sind, umfassend das In-Kontakt-Bringen der Verbindungen mit einem Zell- oder Gewebemodell versprödeter Haut, erhalten durch das Verfahren nach einem der Ansprüche 1 bis 10, **dadurch gekennzeichnet, dass** eine Kandidatenverbindung ausgewählt wird, wenn in ihrer Gegenwart eine Wiederherstellung der Barrierenfunktion beobachtet wird.

## Claims

1. *In vitro* or *ex vivo* method for weakening cells of the epidermis, containing at least two treatment selected from among: a chemical treatment, a dehydrating treatment carried out by placing said cells in a compartment containing a humidity of between 5 and 40% and having a temperature of between 30 and 40°C, a physical treatment and a chemical treatment, **characterised in that** said chemical treatment is carried out by putting into contact said cells with detergents or ketones, **in that** said physical treatment is a UV radiation, and **in that** said mechanical treatment is a compression or a traction.

2. Method according to claim 1, **characterised in that** said chemical treatment is carried out by putting into contact said cells with Sodium Dodecyl Sulphate (SDS).

3. Method according to any one of claims 1 to 2, **characterised in that** said cells are isolated.

4. Method according to claim 3, **characterised in that** said chemical treatment is maintained for a duration of between 30 minutes and 4 hours, preferably 1 hour.

5. Method according to claim 3 or 4, **characterised in that** said dehydrating treatment is maintained for a duration of between 10 minutes and 40 minutes, preferably between 20 and 25 minutes.

6. Method according to any one of claims 1 to 2, **characterised in that** said cells are contained in a tissue, preferably in a reconstructed epidermis.

7. Method according to claim 6, **characterised in that** said chemical treatment is maintained for a duration of between 2 hours and 24 hours, preferably 18 hours.

8. Method according to claim 6 or 7, **characterised in that** said dehydrating treatment is maintained for a duration of between 30 minutes and 2 hours, preferably 1 hour.

9. Method according to any one of claims 1 to 8, **characterised in that** the cells of the epidermis undergo a chemical treatment and a dehydrating treatment.

10. Method according to any one of claims 1 to 9, **characterised in that** said treatments are spaced apart by 4 hours to 30 hours.

11. Screening method for the *in vitro* identification of compounds intended for the care or to the protection of fragile skin, comprising the putting into contact of said compounds with a cell or tissue model of weakened skin obtained by the method according to one of claims 1 to 10, **characterised in that** a candidate compound will be selected if, in the presence thereof, a restoring of the barrier function is observed.
